# EUROPEAN PATENT APPLICATION

(11) **EP 4 485 065 A1**
(43) Date of publication of application: **01.01.2025**
(21) Application number: 23759981.6
(22) Date of filing: 21.02.2023
(51) Int. Cl.: G03B 15/00, G03B 15/02, A61N 5/06, A61B 5/00, G03B 17/48, G03B 17/55

(54) **OPTICAL BEAUTY CARE DEVICE, AND OPTICAL BEAUTY CARE SYSTEM**

(30) Priority: 25.02.2022 JP 2022028612; 25.02.2022 JP 2022028613
(71) Applicant: Panasonic Intellectual Property Management Co., Ltd., Kadoma-shi, Osaka 571-0057 (JP)
(72) Inventor: KASUGAI, Hideki, Kadoma-shi, Osaka 571-0057 (JP); SAKAMOTO, Ryohei, Kadoma-shi, Osaka 571-0057 (JP); KOMATSU, Toshiyuki, Kadoma-shi, Osaka 571-0057 (JP)
(74) Representative: Novagraaf International SA
(86) International application number: PCT/JP2023/006244
(87) International publication number: WO 2023/162980

(57) **Abstract**

The present disclosure is aimed to improve the treatment efficiency to treat the skin. A photocosmetic device includes includes a light emitting unit (2), a housing (1), and a light exit surface (13). The light emitting unit (2) is configured to generate light to be illuminated on a skin (92). The light emitting unit (2) is housed in the housing (1). The light of the light emitting unit (2) is emitted from the light exit surface (13) to an outside of the housing (1). The light exit surface (13) has a recessed shape recessed inward the housing (1). The photocosmetic device further includes a skin introducing unit (8). The skin introducing unit (8) has an opening (800). The skin introducing unit (8) is configured to introduce the skin (92) through the opening (800) into a recessed space (S1) surrounded by the light exit surface (13).

## Description

### Technical Field

The present disclosure relates generally to photocosmetic devices and photocosmetic systems. The present disclosure relates more specifically to a photocosmetic device configured to emit light to be illuminated on the skin, and a photocosmetic system including the photocosmetic device.

### Background Art

Patent Literature 1 describes an optical hair removing apparatus. The optical hair removing apparatus includes an apparatus body having a light source part and a light illumination opening for applying an emission light of the light source part, and a tubular light shielding part installed in a position surrounding the light illumination opening of the apparatus body. A skin contact face formed into a ring shape in a plan view at an end of the light shielding part is defined as a curved face having two projecting portions projecting in a light illumination direction. According to the optical hair removing apparatus of Patent Literature 1, leakage of light is prevented by tightly sticking the light shielding part to a skin surface.

However, in a usual photocosmetic device such as the optical hair removing apparatus of Patent Literature 1, if the light is emitted from the device while the device is located apart from the skin, the light of the device cannot sufficiently reach the inside of the skin due to the light scattering by the skin and the like. This may decline the treatment efficiency for treating the skin.

### Citation List

### Patent Literature

Patent Literature 1: JP 2008-289809 A

### Summary of Invention

The present disclosure is aimed to provide a photocosmetic device and a photocosmetic system having an improved treatment efficiency for treating the skin.

A photocosmetic device according to one aspect of the present disclosure includes a light emitting unit, a housing, and a light exit surface. The light emitting unit is configured to generate light to be illuminated on a skin. The light emitting unit is housed in the housing. The light of the light emitting unit is emitted from the light exit surface to an outside of the housing. The light exit surface has a recessed shape recessed inward the housing. The photocosmetic device further includes a skin introducing unit. The skin introducing unit has an opening. The skin introducing unit is configured to introduce the skin through the opening into a recessed space surrounded by the light exit surface.

A photocosmetic system according to one aspect of the present disclosure includes the photocosmetic device. The photocosmetic device further includes an imaging unit housed in the housing and a communications unit configured to communicate with a terminal device, the terminal device being provided externally. The housing has a recess. The recess of the housing has a bottom surface and at least one side surface from which the light of the light emitting unit is emerged outside. The imaging unit has a light incident surface in the bottom surface of the recess of the housing. The imaging unit is configured to take an image based on light entering through the light incident surface. The communications unit has a function to output, to the terminal device, image information relating to the image taken by the imaging unit. The imaging device is configured to take images a plurality of times at different timings. The photocosmetic system further includes the terminal device. The terminal device has a function to receive, from the communications unit of the photocosmetic device, the image information relating to a plurality of the images taken by the imaging unit at the different timings. The terminal device has a function to display, in time series on a display unit, two or more images selected from the plurality of images represented by the received image information.

### Brief Description of Drawings

FIG. 1 is a sectional view of a main part of a photocosmetic device according to embodiment 1;
FIG. 2 is a front view of the photocosmetic device;
FIG. 3 is a side view of the photocosmetic device when the pinching units are closed;
FIG. 4 is a partially cutaway side view of the photocosmetic device when the pinching units are opened;
FIG. 5 is a schematic view showing a usage state of the photocosmetic device;
FIG. 6 is a block diagram of the photocosmetic device;
FIG. 7 is a sectional view of a main part of the pinching unit of the photocosmetic device;
FIG. 8 is a front view of a main part of the pinching unit;
FIG. 9 is a sectional view of a main part of a photocosmetic device according to a first variation of the embodiment 1;
FIG. 10 is a sectional view of a main part of a photocosmetic device according to a second variation of the embodiment 1;
FIG. 11 is a perspective view of a main part of the photocosmetic device;
FIG. 12 is a perspective view of a main part of a photocosmetic device according to a third variation of the embodiment 1;
FIG. 13 is a perspective view of a main part according to a fourth variation of the embodiment 1;
FIG. 14 is a sectional view of a main part of a first alternative of a pinching unit for the photocosmetic device according to the embodiment 1;
FIG. 15 is a sectional view of a main part of a second alternative of a pinching unit for the photocosmetic device;
FIG. 16 is a sectional view of a main part of a third alternative of a pinching unit for the photocosmetic device;
FIG. 17 is a sectional view of a main part of a fourth alternative of a pinching unit for the photocosmetic device;
FIG. 18 is a sectional view of a main part of a fifth alternative of a pinching unit for the photocosmetic device;
FIG. 19 is a sectional view of a main part of a sixth alternative of a pinching unit for the photocosmetic device;
FIG. 20 is a sectional view of a main part of a seventh alternative of a pinching unit for the photocosmetic device1;
FIG. 21 is a sectional view of a main part of an eighth alternative of a pinching unit for the photocosmetic device;
FIG. 22 is a front view of a ninth alternative of a pinching unit for the photocosmetic device;
FIG. 23 is a front view of a tenth alternative of a pinching unit for the photocosmetic device;
FIG. 24 is a front view of an eleventh alternative of a pinching unit for the photocosmetic device;
FIG. 25 is a front view of a twelfth alterative of a pinching unit for the photocosmetic device;
FIG. 26 is a sectional view of a photocosmetic device according to embodiment 2;
FIG. 27 is a front view of the photocosmetic device;
FIG. 28 is a sectional view of a main part of the photocosmetic device;
FIG. 29 is a sectional view showing a usage state of the photocosmetic device;
FIG. 30 is a block diagram of a photocosmetic system including the photocosmetic device;
FIG. 31 is a perspective view of a main part of a photocosmetic device according to a first variation of the embodiment 2;
FIG. 32 is a block diagram of a photocosmetic system including a photocosmetic device according to a second variation of the embodiment 2;
FIG. 33 is a sectional view of a main part of a photocosmetic device according to a third variation of the embodiment 2;
FIG. 34 is a sectional view of a main part of a photocosmetic device according to a fourth variation of the embodiment 2;
FIG. 35 is a view schematically illustrating a mechanism how the hair falls off from the skin by the illumination of light;
FIG. 36 is a view schematically illustrating a mechanism how the hair falls off from the skin by the illumination of light; and
FIG. 37 is a view schematically illustrating a mechanism how the hair falls off from the skin by the illumination of light.

### Description of Embodiments

Photocosmetic devices according to embodiments will now be described in detail with reference to the accompanying drawings. The drawings to be referred to in the following description of embodiments are all schematic representations. That is to say, the ratio of the dimensions (including thicknesses) of respective constituent elements illustrated on the drawings does not always reflect their actual dimensional ratio. Note that the embodiment and variations to be described below is only an exemplary one of various embodiments of the present disclosure and should not be construed as limiting. Any of the embodiments and the variations can be combined with each other. Moreover, the embodiments and variations may be readily modified in various manners depending on a design choice or any other factor as long as the advantages of the present disclosure are achievable.

### (1) Embodiment 1

### (1.1) Overview of Embodiment 1

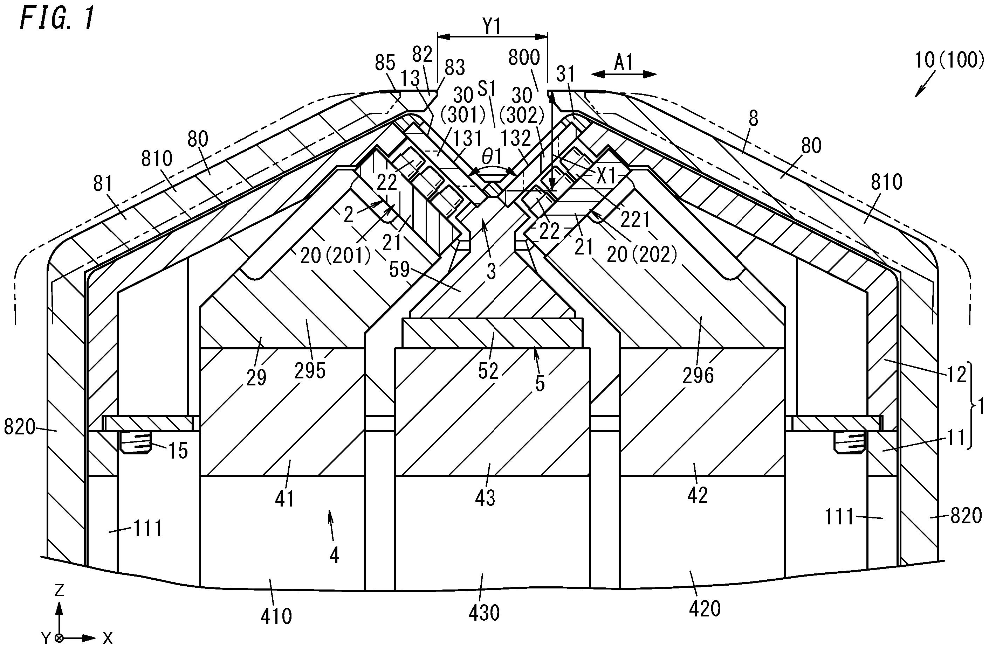

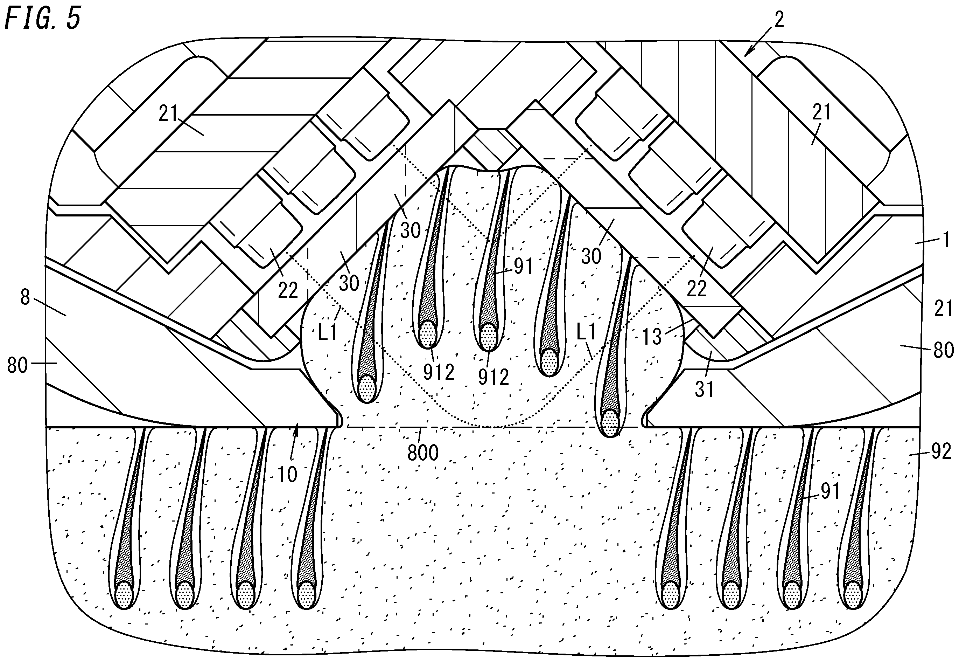

A photocosmetic device 100 of the present embodiment (see FIGS. 1 to 4) is a device for illuminating light to a skin 92 (see FIG. 5) to cause absorption of the light in the skin 92 to treat the skin 92 with the function of the absorbed light.

Specifically, the photocosmetic device 100 of the present embodiment is a body hair treatment device for the hair control or the hair removal, which is configured to emit light to a region of the skin 92 with a hair 91 to facilitate the falling off of the hair 91 from the skin 92. According to the present disclosure, the "hair control" indicates a concept of at least temporary removing the hair 91 from a certain region of the skin 92. That is, the "hair control" tolerates the hair 91 grows up again from the certain region of the skin 92 from which the hair 91 is once removed. According to the present disclosure, the "hair removal" indicates a concept of removing the hair 91 from a certain region of the skin 92 and preventing the hair 91 from growing up again from this region (namely, indicates the permanent hair removal). Accordingly, the photocosmetic device 100 (body hair treatment device) of the present embodiment is a device for the hair control or the hair removal by means of the illumination of the light onto the skin 92.

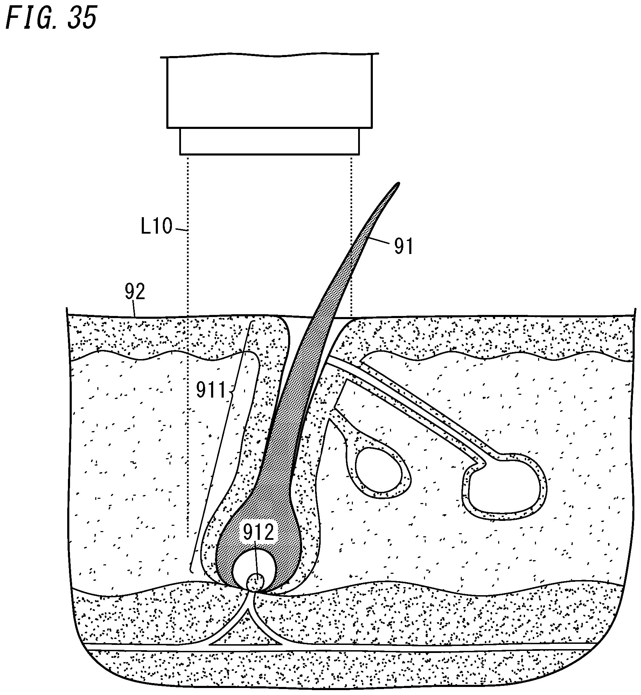

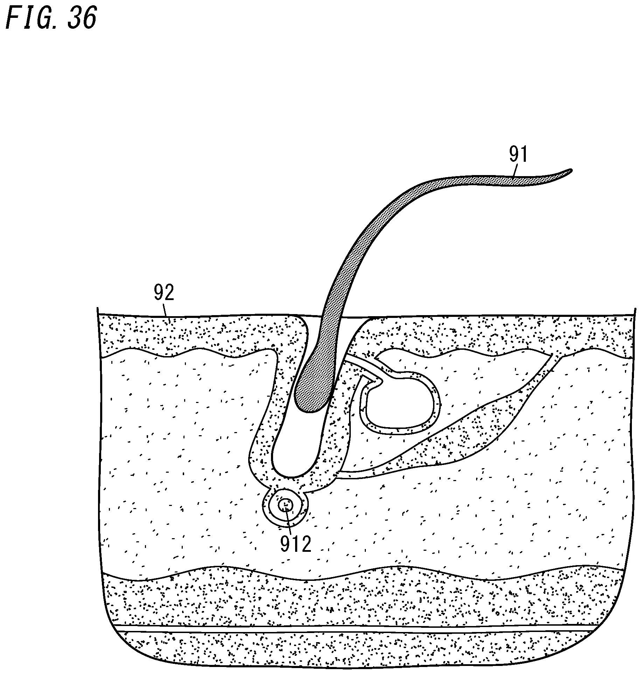

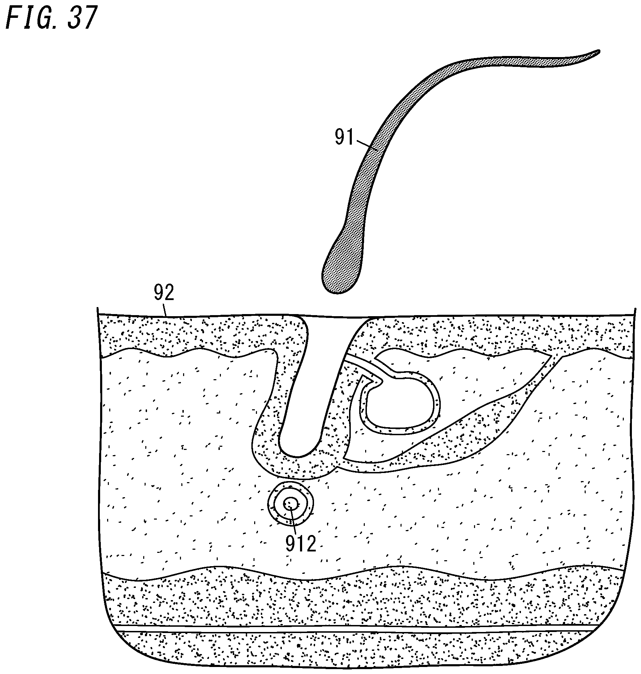

In regard to the hair control or the hair removal by the illumination of the light, the following mechanism is known. Specifically, when a region of the skin 92 with the hair 91 is irradiated with light L10 as shown in FIG. 35, the hair follicle 911 containing the melanin absorbs the irradiated light L10. The hair follicle 911 is heated with the energy of the light L10 thus absorbed, and the hair matrix 912 thereof is damaged with the heat. When the hair matrix 912 is damaged, the hair 91 leaves the hair matrix 912 according to the living body activity (see FIG. 36), and then is fallen off from the skin 92 (see FIG. 37).

Incidentally, around the surface layer of the skin 92 of animals (including the human), there is some substances that may absorb the light L10, such as the water, the vessel, and the like, in addition to the melanin contained in the hair follicle 911. Therefore, when the light L10 is illuminated on the skin 92 for the purpose of e.g., the hair control or the hair removal, at least part of the light L10 is absorbed in such the substances (water etc.). When the large amount (proportion) of the light L10 is absorbed in the substances such as the water in the skin 92, the hair matrix 912 may not be damaged sufficient to cause the falling off of the hair 91. This may decline the treatment efficiency (hair control efficiency, hair removal efficiency) of the hair 91 (body hair).

In this regard, according to the photocosmetic device 100 of the present embodiment, a light exit surface 13 from which light of a light emitting unit 2 is emitted to an outside of a housing 1 has a recessed shape recessed inward the housing 1, as shown in FIG. 1. Moreover, the photocosmetic device 100 of the present embodiment includes a skin introducing unit 8. The skin introducing unit 8 has an opening 800 and is configured to introduce the skin 92 into a recessed space S1 surrounded by the light exit surface 13 through the opening 800.

According to the photocosmetic device 100, therefore, the skin introducing unit 8 can introduce the skin 92 into the recessed space S1 to cause the skin 92 to be brought into (close) contact with the light exit surface 13, as shown in FIG. 5. This can uniquely set a positional relation between the light emitting unit 2 and the skin 92 even in a repeated use (i.e., without fluctuating the distances in each use). As a result, the light of the light emitting unit 2 can be illuminated on a desired region (e.g., a region of the skin 92 corresponding to the hair matrix 912) of the skin 92 even in a repeated use. This can efficiently provide the damage to the hair matrix 912 to improve the skin treatment efficiency.

### (1.2) Details of Embodiment 1

The photocosmetic device 100 of the present embodiment is described in detail below with reference to FIGS. 1 to 8. The photocosmetic device 100 of the present embodiment is the body hair treatment device for treating the body hair (hair 91) on the skin 92, as described above.

The following description is made based on one example where three axes are defined which are perpendicular to one another, namely the X-axis (first axis), the Y-axis (second axis) and the Z-axis (third axis). Specifically, the "X-axis" is defined as an axis along which a plurality of light source units 20 included in the light emitting unit 2 are arranged, and the "Z-axis" is defined as an axis along an opening direction of the recessed space S1. According to the present disclosure, the "opening direction" indicates a normal direction of an imaginal plane containing the opening 800. Note that the X-axis, the Y-axis and the Z-axis are all imaginary, and the arrows in the figures with the characters of "X", "Y", and "Z" are illustrated for the explanation purpose only and are not tangible.

In the description below, the positive and negative sides in the X-axis will be referred to as the "right" and "left", respectively, and the positive and negative sides in the Z-axis will be referred to as the "front" and "rear", respectively. However, these definitions of the directions do not limit the orientation of the photocosmetic device 100 when it is used.

According to the present disclosure, the "hair (body hair)" includes various hairs 91 protruding from the skin 92, namely various hairs on the skin 92, and may include various body hairs of the human such as hair on the head, beard or mustache, eyebrow, leg hair, nose hair, ear hair and the like. Moreover, the "hair (body hair)" used herein may include various hairs 91 protruding from the skin 92 of mammals such as dog or cat, or other animals. That is, the irradiation target of the body hair treatment device as an example of the photocosmetic device 100 of the present embodiment includes various skins 92 from which the hair 91 protrudes.

According to the present disclosure, the "user" indicates a subject who has the skin 92 to be irradiated with the light L1 from the photocosmetic device 100. The "user" is, for example, an animal including humans, mammals, and other animals. If the user is a human, the photocosmetic device 100 may be operated (held) by the user oneself, or may be operated by a person other than the user. If the user is an animal other than the human, the photocosmetic device 100 will be usually operated by a person other than the user.

In the following, a hair control device 10 will be described as an example of the photocosmetic device 100 (body hair treatment device) of the present embodiment.

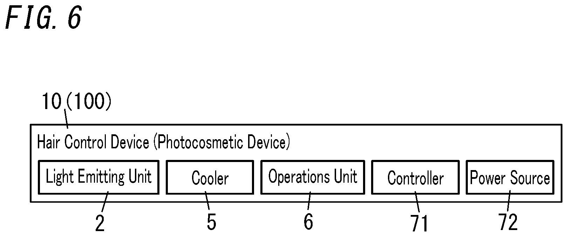

As shown in FIGS. 1, 6, the hair control device 10 includes the housing 1, the light exit surface 13, the light emitting unit 2, a light transmission member 3, a heat dissipation part 4, a cooler 5, an operations unit 6, a controller 71, a power source 72, and the skin introducing unit 8.

The housing 1 houses therein the light emitting unit 2, the light transmission member 3, the heat dissipation part 4, the cooler 5, the controller 71, and the power source 72. The operations unit 6 is provided to an outer peripheral surface of the housing 1, for example. The skin introducing unit 8 is held by the housing 1.

As shown in FIGS. 1 to 4, the housing 1 includes a first case 11, a second case 12 and a third case 17.

The first case 11 is made from resin, for example. The first case 11 has a tubular shape opened forward and rearward. The first case 11 has a side wall provided with a plurality of air vents 111 (see FIG. 1) penetrating the side wall.

The second case 12 is made from resin, for example. The second case 12 is disposed on the front side of the first case 11 to cover a front opening of the first case 11. The second case 12 is coupled to the first case 11 with screws 15.

The third case 17 is made from resin, for example. The third case 17 has a rectangular tube shape opened forward and rearward. The third case 17 is a bit smaller than the first case 11 when viewed in the Z-axis. The third case 17 is disposed on the rear side of the first case 11 such that a front opening of the third case 17 is continuous with a rear opening of the first case 11. The third case 17 and the first case 11 are coupled together with screws.

The light exit surface 13 is a surface from which the light of the light emitting unit 2 is emitted to the outside of the housing 1. According to the hair control device 10 of the present embodiment, the light exit surface 13 is positioned in a front surface of the housing 1 (a front surface of the second case 12).

As shown in FIG. 1, the light exit surface 13 has a recessed shape recessed inward the housing 1. The light exit surface 13 includes a first inclined surface 131 and a second inclined surface 132. The first inclined surface 131 and the second inclined surface 132 face each other in the X-axis. The first inclined surface 131 and the second inclined surface 132 cross each other at a crossing angle θ1. The crossing angle θ1 is set to have an angle value falling within the range greater than 0° and smaller than 180°. The crossing angle θ1 may have an angle value falling within the range equal to or greater than 30° and equal to or smaller than 120°. The crossing angle θ1 may have an angle value falling within the range equal to or greater than 30° and equal to or smaller than 60°. The crossing angle θ1 may have an angle value falling within the range equal to or greater than 60° and equal to or smaller than 120°. The crossing angle θ1 may have an angle value falling within the range equal to or greater than 60° and equal to or smaller than 80°. The crossing angle θ1 may have an angle value falling within the range equal to or greater than 80° and equal to or smaller than 120°. The crossing angle θ1 may have an angle value falling within the range equal to or greater than 90° and equal to or smaller than 120°. As one example, according to the hair control device 10 of the present embodiment, the crossing angle θ1 is 90°.

As shown in FIG. 1, the recessed space S1 is defined as a space surrounded by the light exit surface 13 (between the first inclined surface 131 and the second inclined surface 132). The recessed space S1 is a space into which the skin 92 to be treated is introduced. The recessed space S1 is continuous with an outside space through the opening 800 of the skin introducing unit 8. According to the hair control device 10 of the present embodiment, the recessed space S1 includes a V-shaped groove space surrounded by the light exit surface 13 when viewed in the Y-axis. A bottom angle of the groove of the recessed space S1 is the same as the crossing angle θ1. When the bottom angle of the groove of the recessed space S1 is greater than 0° and smaller than 180°, the skin 92 can be introduced into the recessed space S1 by virtue of the frictional force between the skin 92 and the skin introducing unit 8. When the bottom angle of the groove of the recessed space S1 falls within the range equal to or greater than 30° and equal to or smaller than 120°, the surface of the skin 92 can be easily positioned near the light exit surface 13. According to the bottom angle within this range, the amount of the light illuminated from the first inclined surface 131 and the second inclined surface 132 can be increased at the hair follicle 911 by 1.8 times to 4.5 times compared to the conventional planar light irradiation (corresponding to a case where θ1= 180°). The irradiation efficiency can be increased. Moreover, when the bottom angle of the groove of the recessed space S1 falls within the range equal to or greater than 80° and equal to or smaller than 120°, the amount of the light can be increased at the hair follicle 911 by 1.8 times to 2.5 times compared to the planar light irradiation to increase the effect accordingly. Furthermore, when the bottom angle of the groove of the recessed space S1 falls within the range equal to or greater than 60° and equal to or smaller than 80°, the relation between the first inclined surface 131 and the second inclined surface 132 approaches the near parallel state, and the amount of the light illuminated from the two light source units 20 can be increased at the hair follicle 911 by 3.4 times at the maximum compared to the planar light irradiation to increase the effect accordingly. The bottom angle may preferably fall within the range equal to or greater than 30° and equal to or smaller than 60°, which can increase the amount of the light by 4.5 times at the maximum to increase the effect accordingly.

As shown in FIG. 1, the light emitting unit 2 includes a plurality of (two, in the embodiment) light source units 20, and a holding member 29.

As shown in FIG. 1, each of the plurality of light source units 20 includes a substrate 21 and a light source 22.

The substrate 21 has a rectangular plate shape. The substrate 21 extends along the Y-axis.

The light source 22 is mounted on the substrate 21. The light source 22 is mounted on a surface on the front side of the substrate 21.

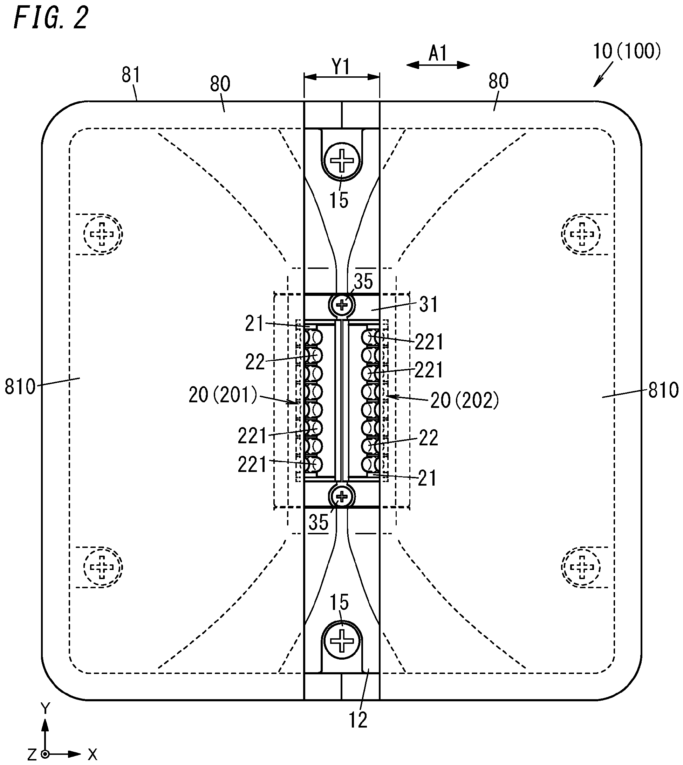

As shown in FIGS. 1, 2, the light source 22 includes a plurality of light emitting diodes (LEDs) 221. In the LED 221, an LED device (semiconductor device) is enclosed in a case made from resin. That is, the light emitting unit 2 includes a plurality of LEDs.

The LED 221 has an emission wavelength falling within the range from 600 nm to 1000 nm. The LED 221 is a red LED or a near-infrared LED. An example of the emission wavelength of the LED 221 is about 850 nm. Another example of the emission wavelength of the LED 221 is about 950 nm. According to the present disclosure, the feature "emission wavelength of the LED 221" indicates a peak emission wavelength of the LED 221, that is, a wavelength exhibiting the maximum intensity in an emission spectrum of the LED 221.

The LED 221 is a monochromatic light source configured to emit a monochromatic light. That is, the LED 221 is configured to emit light having a relatively narrow bandwidth. The bandwidth of the emission light of the LED 221 may fall within the range from 60 nm to 100 nm, for example. According to the present disclosure, the feature "bandwidth of the emission light of the LED 221" indicates a full width at half maximum of the spectrum of the emission light of the LED 221.

The plurality of LEDs 221 have the same emission wavelengths as one another. According to the present disclosure, the feature "the plurality of LEDs 221 have the same emission wavelengths as one another" is not limited to a condition where the emission wavelengths (peak emission wavelengths) of the plurality of LEDs 221 are exactly identical to one another, but accepts a certain degree of tolerance such as the manufacturing errors, for example.

As shown in FIG. 2, the plurality of LEDs 221 are arranged in an array on the substrate 21. That is, the light source 22 includes an LED array including the plurality of LEDs 221 arranged in an array. In the embodiment, the light source 22 includes 24-pieces of LEDs 221. The 24-pieces of LEDs 221 are arranged in a 3x8 array viewed from the front, where there are three pieces along the X-axis and eight pieces along the Y-axis.

Each of the plurality of LEDs 221 is mounted on the substrate 21 such that it exhibits a light distribution property where the direction of the peak intensity is aligned with the normal direction of the substrate 21. In other words, each of the plurality of LEDs 221 has an optical axis along the normal direction of the substrate 21 on which the LED 221 is mounted.

In each light source unit 20, the light source 22 has an optical axis along the normal direction of the substrate 21. According to the present disclosure, the feature "optical axis of the light source 22 including the plurality of LEDs 221" indicates an imaginary axis (pillar shaped axis) defined by a region surrounded by the optical axes of the plurality of LEDs 221.

According to the hair control device 10 of the present embodiment, each of the plurality of light source units 20 has an optical axis. The optical axis of the light source unit 20 indicates an imaginary line representing the ray of light emitted from the light source unit 20. According to the hair control device 10 of the present embodiment, the optical axis of the light source unit 20 coincides with the optical axis of the light source 22. The optical axis of the light source unit 20 is along the normal direction of the substrate 21.

Hereinafter, the two light source units 20 may be referred to as a first light source unit 201 and a second light source unit 202, respectively, in order to distinguish the two light source units 20 from each other. As shown in FIG. 1, the first light source unit 201 is one of the two light source units 20 located on the left side (on the negative side in the X-axis). The second light source unit 202 is the other of the two light source units 20 located on the right side (on the positive side in the X-axis).

The substrate 21 of the first light source unit 201 is parallel to the first inclined surface 131 of the light exit surface 13. In the X-Z plane, the substrate 21 of the first light source unit 201 is inclined with respect to the X-axis by a predetermined angle (in the embodiment, 135°). The light L1 (see FIG. 5) of the first light source unit 201 is emitted to an outside of the housing 1 (specifically, to the recessed space S1) from the first inclined surface 131 of the light exit surface 13.

The substrate 21 of the second light source unit 202 is parallel to the second inclined surface 132 of the light exit surface 13. In the X-Z plane, the substrate 21 of the second light source unit 202 is inclined with respect to the X-axis by a predetermined angle (in the embodiment, 45°). The light L1 of the second light source unit 202 is emitted to the outside of the housing 1 (specifically, to the recessed space S1) from the second inclined surface 132 of the light exit surface 13.

The holding member 29 has the heat conduction property. The holding member 29 may be made of metal or ceramic, for example.

The holding member 29 holds the substrates 21 of the plurality of (two) light source units 20. The holding member 29 holds the substrates 21 of the plurality of light source units 20 such that the plurality of (two) light source units 20 are arranged along the X-axis. As shown in FIG. 2, the plurality of (two) light source units 20 are arranged along the X-axis with their short-side axes of the respective substrates 21 are aligned each other when viewed from the front side.

The holding member 29 includes a first holding member 295 holding the first light source unit 201 and a second holding member 296 holding the second light source unit 202.

The first light source unit 201 is disposed on a front surface of the first holding member 295. The front surface of the first holding member 295 is parallel to the first inclined surface 131 of the light exit surface 13.

The second light source unit 202 is disposed on a front surface of the second holding member 296. The front surface of the second holding member 296 is parallel to the second inclined surface 132 of the light exit surface 13.

The plurality of light source units 20 (the first light source unit 201 and the second light source unit 202) are held by the holding member 29 such that the optical axes of the plurality of light source units 20 cross one another on the outside of the housing 1. According to the hair control device 10 of the present embodiment, the optical axes of the plurality of light source units 20 (the first light source unit 201 and the second light source unit 202) cross each other inside the recessed space S1.

According to the hair control device 10 of the present embodiment, as shown in FIG. 5, the plurality of light source units 20 (the first light source unit 201 and the second light source unit 202) are arranged such that the rays of the light L1 of the plurality of light source units 20 cross each other in a region around the hair matrix 912 (bulb) in the skin 92 in a state where the surface of the skin 92 is in contact with the light exit surface 13 (i.e., in a state where the skin 92 is introduced into the recessed space S1). As one example, the first light source unit 201 and the second light source unit 202 are arranged such that the rays of the light L1 of the first light source unit 201 and the second light source unit 202 cross each other at a point where a distance from the first inclined surface 131 to the point falls within the range from about 2 mm to 5 mm (e.g., about 4 mm) and also a distance from the second inclined surface 132 to the point falls within the range from about 2 mm to 5 mm (e.g., about 4 mm). A crossing angle of the light source units 20 (i.e., a crossing angle of the optical axes thereof) are determined in consideration with the refractive index of the skin 92 and the like such that the rays of the light L1 of the plurality of light source units 20 (the first light source unit 201 and the second light source unit 202) cross each other in the region around the hair matrix 912 in the skin 92 in a state where the surface of the skin 92 is in contact with the light exit surface 13. A crossing angle between two light source units 20 (crossing angle of the optical axes) having largest angle value among the crossing angles of the plurality of (two) light source units 20 is set to fall within the range equal to or greater than 10° and equal to or smaller than 180°, for example. When the two light source units 20 are arranged such that the rays of the light L1 cross each other in the skin 92 at the depth from the surface of the skin 92 falling within the range from about 2 mm to about 5 mm (e.g., about 4 mm) with the crossing angle less than 10°, the light sources 22 of the two light source units 20 can include only a small number of LEDs 221. This may decrease the amount of light of the light sources 22. The crossing angle of 180° or more may cause the difficulty to deform the skin 92 along the light exit surface 13 having the recessed shape. Moreover, for realizing the optical axes crossing each other in the inside of the skin 92 with a large crossing angle, the light source units 20 should be disposed apart from each other, which may increase the size of the device. The crossing angle may be smaller than 170°. The crossing angle may fall within the range equal to or greater than 50° and equal to or smaller than 160°. The crossing angle may fall within the range equal to or greater than 90° and equal to or smaller than 150°.

In an example, the light emitted from the light emitting unit 2 (i.e., the plurality of light source units 20) has the intensity such that the light density on the light exit surface 13 falls within the range from 15 W/cm² to 35 W/cm². The emission duration of the light emitted from the light emitting unit 2 falls within the range from 500 ms to 2000 ms, for example. The light having the intensity within the above range with the emission duration equal to or greater than 500 ms can provide a high effect on the hair 91 which is in a growth stage of an early stage of the anagen to the anagen. Moreover, the light having the intensity within the above range with the emission duration equal to or smaller than 2000 ms can advantageously suppress the excessive temperature increase in the skin 92 caused by the absorption of the light.

The light transmission member 3 is disposed in front of the light emitting unit 2. The light exit surface 13 is at least partially constituted by a surface of the light transmission member 3. In the present embodiment, the surface (front surface) of the light transmission member 3 on the outer side of the housing 1 constitutes (a whole of) the light exit surface 13. The light transmission member 3 allows the light of the light sources 22 of the plurality of light source units 20 of the light emitting unit 2 to pass through. Note that the illustration of the light transmission member 3 is omitted in FIG. 2.

The light transmission member 3 is made of the material having the small absorptance at the wavelength of the light emitted from the light source 22. In other words, the light transmission member 3 is made of the material exhibiting small absorption loss with respect to the light of the light source 22. The light transmission member 3 is transparent at the wavelength of the light of the light source 22. The light transmission member 3 is made of the material having the small extinction coefficient at the wavelength of the light of the light source 22. It may be preferable that the light transmission member 3 is made of the material having the extinction coefficient of 0.0 at the wavelength of the light of the light source 22. The light transmission member 3 having the small absorptance of the light is less likely to absorb the light, which can suppress the increase in the temperature of the light transmission member 3. The skin 92 in contact with the light transmission member 3 will be less stimulated.

Furthermore, the light transmission member 3 is made of the material having the refractive index greater than that of the skin 92 of the human at the wavelength of the light of the light source 22 (i.e., at the emission wavelength of the light source 22). In the embodiment, the light transmission member 3 is made of the material having the refractive index of 1.7 or more at the emission wavelength of the light source 22. The refractive index is measured in compliance with the measuring method for refractive index of optical glass, defined by JIS B 7071, for example. The light transmission member 3 having the refractive index greater than the refractive index of the skin 92 can reduce the total reflection of the light at the boundary between the light transmission member 3 and the surface of the skin 92 when the light travels in the light transmission member 3 toward the skin 92.

The light transmission member 3 has the heat conduction property. The light transmission member 3 is made of the material having the thermal conductivity of 1 W/mK or more. The thermal conductivity is measured in compliance with the measurement method of thermal conductivity for fine ceramics by flash method, defined by JIS R 1611, for example. The light transmission member 3 having the heat conduction property can facilitate the cooling of the body in contact with the light transmission member 3.

The light transmission member 3 is made of the material having the refractive index of 1.7 or more at the wavelength of the light of the light source 22 and having the thermal conductivity of 1 W/mK or more, for example. Specifically, the light transmission member 3 is made of at least one material selected from the group consisting of sapphire (Al₂O₃), zinc oxide (ZnO), zirconium oxide (ZrO₂), magnesium oxide (MgO), gallium nitride (GaN), aluminum nitride (AIN), and diamond. The light transmission member 3 may preferably be made of sapphire. The light transmission member 3 may be made of two or more materials. The refractive indices and the thermal conductivities (W/mK) of these materials are shown in the following Table 1.

The refractive index shown in the Table 1 is a value at a particular wavelength (specifically, d-line: 587.6 nm). Note that the value of the refractive index of the material varies depending on the wavelength of the light of the light source 22, due to the wavelength dispersion of the refractive index. For example, the value of the refractive index of the sapphire decreases as the increase in the wavelength. Specifically, the refractive index of the sapphire at the wavelength of 850 nm is 1.76, for example. The value of the refractive index of the sapphire increase as the decrease in the wavelength. The refractive index of the sapphire at the wavelength of 405 nm is 1.786, for example.

**Table 1**

| | Refractive Index | Thermal Conductivity (W/mK) |
|---|---|---|
| Sapphire | 1.768 | 42 |
| ZnO | 2.01 | 20 |
| ZrO₂ | 2.217 | 3 |
| MgO | 1.74 | 47 |
| GaN | 2.393 | 200 |
| AlN | 2.175 | 150 |
| Diamond | 2.417 | 1000 |

As shown in FIG. 1, the light transmission member 3 includes a plurality of (two) light transmission pieces 30. Each of the plurality of light transmission pieces 30 has a plate shape. The plurality of light transmission pieces 30 are arranged to correspond (one-to-one) to the plurality of light source units 20. Each of the plurality of light transmission pieces 30 is disposed to face the light source 22 (a plurality of LEDs 221) of a corresponding light source unit 20 to increase the amount of light. That is, the light sources 22 of the light emitting unit 2 (light source units 20) are arranged to face the light transmission member 3 (light transmission pieces 30). This can reduce the distance from the light source 22 to the light exit surface 13 compared to a case where the light source 22 does not face the light transmission member 3.

Hereinafter, in order to distinguish the two light transmission pieces 30 from each other, a light transmission piece 30 corresponding to the first light source unit 201 may be referred to as a first light transmission piece 301, and a light transmission piece 30 corresponding to the second light source unit 202 may be referred to as a second light transmission piece 302, respectively.

A front surface of the first light transmission piece 301 serves as the first inclined surface 131 of the light exit surface 13, and a front surface of the second light transmission piece 302 serves as the second inclined surface 132 of the light exit surface 13. That is, the light exit surface 13 includes the front surface of the first light transmission piece 301 (the first inclined surface 131) and the front surface of the second light transmission piece 302 (the second inclined surface 132). The front surface of the first light transmission piece 301 and the front surface of the second light transmission piece 302 form the bottom of the V-shaped groove of the recessed space S1.

According to the hair control device 10 of the present embodiment, a depth of the recessed space surrounded by the light exit surface 13 falls within the range of greater than 0 mm and equal to or smaller than 30 mm. According to the present disclosure, the feature "depth of the recessed space surrounded by the light exit surface 13" indicates a depth of the surface, from which the light is to be emitted, of the housing 1. In one specific example, the "depth of the recessed space surrounded by the light exit surface 13" indicates the dimension measured along the Z-axis of the first inclined surface 131 (or the second inclined surface 132). The depth of the recessed space surrounded by the light exit surface 13 may preferably fall within the range of equal to or greater than 2 mm and equal to or smaller than 30 mm.

According to the hair control device 10 of the present embodiment, a depth X1 falls within the range greater than 0 mm and equal to or smaller than 30 mm. The depth X1 is measured as a distance between the opening 800 and a far end part, most distant from the opening 800, of the light exit surface 13. In a specific example, the feature "the depth X1 measured as a distance between the opening 800 and a far end, most distant from the opening 800, of the light exit surface 13" indicates a dimension measured along the Z-axis between the opening 800 and the far end part of the light exit surface 13, which is the most distant part in the Z-axis of the light exit surface 13 from the opening 800 (see FIG. 1). The depth X1 may preferably fall within the range of equal to or greater than 2 mm and equal to or smaller than 30 mm.

The depth X1 equal to or greater than the lower limit of the above range allows the skin 92 to be sufficiently introduced into the recesses space S1, which can shorten the treatment time. The depth X1 greater than the upper limit of the above range may cause the difficulty in deforming the skin 92 along the light exit surface 13.

The front surface of each light transmission piece 30 intersects with the optical axis of the corresponding light source unit 20. In the embodiment, the front surface of each light transmission piece 30 intersects with the optical axis of the corresponding light source unit 20 at right angles. Specifically, the front surface of the first light transmission piece 301 (i.e., the first inclined surface 131) intersects with the optical axis of the first light source unit 201 (at right angles, in the embodiment). The front surface of the second light transmission piece 302 (i.e., the second inclined surface 132) intersects with the optical axis of the second light source unit 202 (at right angles, in the embodiment). In short, according to the hair control device 10 of the present embodiment, the optical axis of the light emitting unit 2 intersects with the light exit surface 13 (at right angles, in the embodiment).

The light transmission member 3 is held by a holder 31. According to the hair control device 10 of the present embodiment, the holder 31 has a V-shaped plate shape when viewed laterally, and has openings into which the first light transmission piece 301 and the second light transmission piece 302 are respectively fitted. The holder 31 is fixed to a coupling member 59 (described later) with screws 35 (see FIG. 2).

According to the hair control device 10 of the present embodiment, the surface (front surface) of the light transmission member 3 constitutes the light exit surface 13. The hair control device 10 is adapted to be used in a state where the front surface of the light transmission member 3 (i.e., the light exit surface 13) is in contact with the skin 92 introduced into the recesses space S1 (see FIG. 5). In the usage state of the hair control device 10, therefore, the skin 92 which is in contact with the front surface of the light transmission member 3 (i.e., the light exit surface 13) is cooled by the heat of the skin 92 being taken away by the light transmission member 3. That is, the light transmission member 3 has a cooling capability for facilitating the cooling of the skin 92 in contact with the light transmission member 3. The light transmission member 3 having the cooling capability can suppress the increase in the temperature of the skin 92 around the surface layer. The nerves that detect the pain in the skin 92 will be less stimulated, and the uncomfortable feeling to be caused in a user can be reduced accordingly. In short, the hair control device 10 of the present embodiment includes a cooling member (light transmission member 3) disposed to be brought into contact with the skin 92 to cool the skin 92.

The cooler 5 is disposed to cool the light transmission member 3 (the plurality of light transmission pieces 30). The cooler 5 is disposed in the housing 1.

According to the present embodiment, the cooler 5 includes a Peltier device 52. The Peltier device 52 is disposed in the second case 12. The Peltier device 52 is disposed such that the "cold" side is directed forward.

The coupling member 59 is disposed on a front surface of the Peltier device 52. The coupling member 59 has the heat conduction property. The coupling member 59 is made of metal, for example.

As shown in FIG. 1, the coupling member 59 is coupled to the holder 31 such that the light transmission member 3 (the plurality of light transmission pieces 30) is in contact with the coupling member 59. The front surface of the Peltier device 52 is thermally connected to the light transmission member 3 (the plurality of light transmission pieces 30) via the coupling member 59.

The cooler 5 (Peltier device 52) can facilitate the cooling of the light transmission member 3 (the plurality of light transmission pieces 30), which can suppress the increase in the temperature of the skin 92 around the surface layer in contact with the light transmission member 3. The uncomfortable feeling to be caused in a user can be reduced.

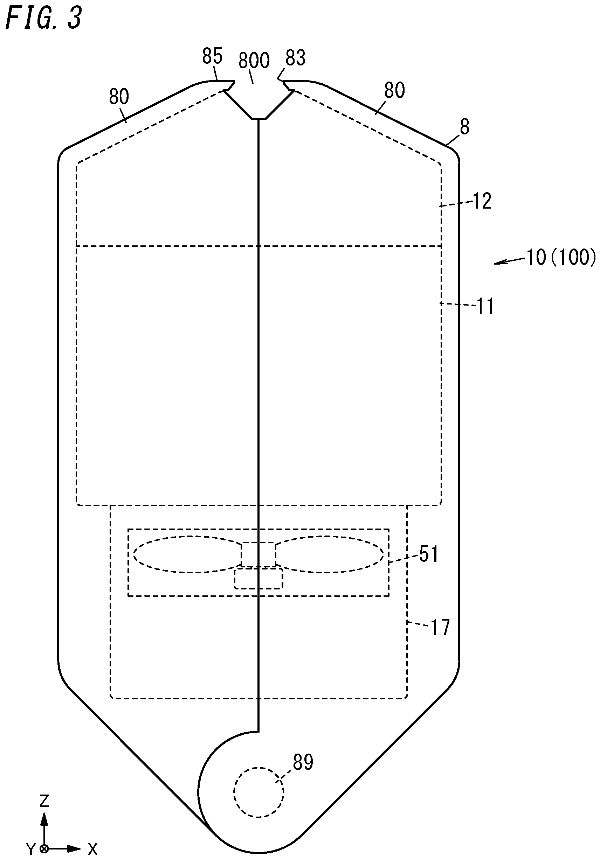

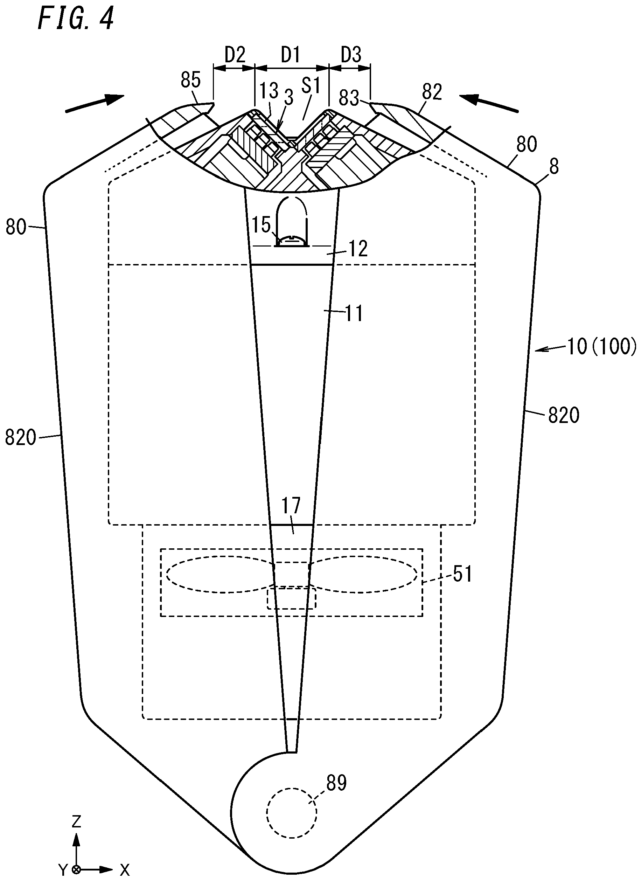

As shown in FIGS. 3, 4, the cooler 5 further includes a cooling fan 51. The cooling fan 51 is disposed in the third case 17. The cooling fan 51 is disposed in the housing 1 and configured to send out the wind forward (flowing to the positive side in the Z-axis).

The wind sent out from the cooling fan 51 reaches a first heat dissipation part 41 to a third heat dissipation part 43 (described later) of the heat dissipation part 4 to take the heat away from the first heat dissipation part 41 to the third heat dissipation part 43. This can facilitate the cooling of the first heat dissipation part 41 to the third heat dissipation part 43.

The cooler 5 (the cooling fan 51 and the Peltier device 52) is controlled by the controller 71 such that the temperature of the surface of the skin 92 in contact with the light transmission member 3 falls within a predetermined temperature range, for example. The predetermined temperature range is a range of -5°C to 35°C, for example. Making the temperature of the surface of the skin 92 equal to or greater than -5°C can reduce the uncomfortable feeling due to the low temperature state of the skin 92. Making the temperature of the surface of the skin 92 equal to or smaller than 35°C can reduce the uncomfortable feeling due to the high temperature state of the skin 92.

The hair control device 10 may include a temperature sensor configured to measure the temperature of the skin 92. The controller 71 may be configured to operate the cooler 5 based on the measurement result of the temperature sensor. It should be noted that the hair control device 10 can make the temperature of the surface of the skin 92 fall within the above predetermined temperature range without the temperature sensor by, for example, preliminary setting the operation condition of the cooler 5 appropriately or allowing the user to turn the cooler 5 on and off by a switch and the like.

As shown in FIG. 1, the heat dissipation part 4 includes a first heat dissipation part 41, a second heat dissipation part 42, and a third heat dissipation part 43. The first heat dissipation part 41 is coupled to the first holding member 295. The first heat dissipation part 41 is designed to primarily dissipate the heat of the first light source unit 201. The second heat dissipation part 42 is coupled to the second holding member 296. The second heat dissipation part 42 is designed to primarily dissipate the heat of the second light source unit 202. The third heat dissipation part 43 is coupled to the Peltier device 52 of the cooler 5. The third heat dissipation part 43 is designed to primarily dissipate the heat of the Peltier device 52.

The first heat dissipation part 41 has the heat conduction property. The first heat dissipation part 41 is made of metal, for example. The first heat dissipation part 41 includes a plurality of first heat dissipation fins 410. Each of the plurality of first heat dissipation fins 410 has a plate shape extending along the Z-axis. Respective front ends of the plurality of first heat dissipation fins 410 are coupled together. The plurality of first heat dissipation fins 410 are disposed in an inside space of the first case 11. The plurality of first heat dissipation fins 410 are arranged along the Y-axis. A gap is formed between two first heat dissipation fins 410 adjacent to each other in the Y-axis.

The second heat dissipation part 42 has the heat conduction property. The second heat dissipation part 42 is made of metal, for example. The second heat dissipation part 42 includes a plurality of second heat dissipation fins 420. Each of the plurality of second heat dissipation fins 420 has a plate shape extending along the Z-axis. Respective front ends of the plurality of second heat dissipation fins 420 are coupled together. The plurality of second heat dissipation fins 420 are disposed in the inside space of the first case 11. The plurality of second heat dissipation fins 420 are arranged along the Y-axis. A gap is formed between two second heat dissipation fins 420 adjacent to each other in the Y-axis.

The third heat dissipation part 43 has the heat conduction property. The third heat dissipation part 43 is made of metal, for example. The third heat dissipation part 43 includes a plurality of third heat dissipation fins 430. Each of the plurality of third heat dissipation fins 430 has a plate shape extending along the Z-axis. Respective front ends of the plurality of third heat dissipation fins 430 are coupled together. The plurality of third heat dissipation fins 430 are disposed in the inside space of the first case 11. The plurality of third heat dissipation fins 430 are arranged along the Y-axis. A gap is formed between two third heat dissipation fins 430 adjacent to each other in the Y-axis.

The first heat dissipation fin 410, the second heat dissipation fin 420, and the third heat dissipation fin 430 are located at the same position as one another in the Y-axis. Therefore, the gap of the first heat dissipation fins 410, the of the second heat dissipation fins 420, and the gap of the third heat dissipation fins 430 are located at the same position as one another in the Y-axis.

The operations unit 6 is configured to be operated by the user. The operations unit 6 includes a manual switch provided on the outer peripheral surface of the housing 1, for example.

The controller 71 is configured to control the operation of the hair control device 10. The controller 71 is configured to control the operations of the light emitting unit 2 and the cooler 5.

The controller 71 is implemented as a microcontroller including one or more processors and one or more memories. The microcontroller performs the function of the controller 71 by making the one or more processors execute a program stored in the one or more memories. The program may be stored in advance in the memories, or may be downloaded via a telecommunications line or distributed after having been stored in a non-transitory storage medium such as a memory card. In other words, the program is a program for making the one or more processors function as the controller 71.

The controller 71 controls the operations of the light emitting unit 2 and the cooler 5 (Peltier device 52) in accordance with the operation given to the operations unit 6, for example. The controller 71 is configured to make the light emitting unit 2 start emitting the light and make the cooler 5 start operating in accordance with the operation given to the operations unit 6, for example. The controller 71 is configured to, in accordance with another operation given to the operations unit 6, stop the light emitting unit 2 emitting the light, and then stop the cooler 5 operating after a predetermined time (which may be 0 second), for example.

The power source 72 supplies the electric power to the light emitting unit 2, the cooler 5 and the controller 71. The power source 72 supplies the DC power to the light emitting unit 2, the cooler 5 and the controller 71. The power source 72 may be a battery, for example. The power source 72 may include a rechargeable secondary battery and a charging circuit configured to convert an AC power supplied from an external AC power supply into a DC power to charge the secondary battery. However, the power source 72 is not limited thereto, but may include a power source circuit including an AC/DC converter configured to convert an AC power supplied from an external AC power supply into a DC power.

The skin introducing unit 8 is configured to introduce the skin 92 into the recessed space S1. The skin introducing unit 8 has the opening 800, and is configured to introduce the skin 92 into the recessed space S1 through the opening 800.

According to the hair control device 10 of the present embodiment, the skin introducing unit 8 includes a pinching unit 80. The pinching unit 80 is made from resin, for example.

According to the hair control device 10 of the present embodiment, the skin introducing unit 8 includes a pair of pinching units 80. As shown in FIGS. 1, 2, the pair of pinching units 80 are arranged such that the pair of pinching units 80 cover side surfaces (a left side surface and a right side surface) and a front surface (i.e., a front surface of the second case 12) of the housing 1 in a cross section perpendicular to the Y-axis and taken along a plane containing the light emitting unit 2 and the light transmission member 3. On a position in front of the housing 1, the pair of pinching units 80 face each other in the X-axis.

Specifically, each the pinching unit 80 includes: a front wall 810 located in front of the front surface of the housing 1 (front surface of the second case 12); and three side walls 820 cover the side surfaces of the housing 1 from three directions. A front wall 810 of one of the pair of pinching units 80 (refereed to as a "first pinching unit 80") covers the substantially left half of the front surface of the housing 1. Three side walls 820 of the first pinching unit 80 cover the left side of the housing 1 and also cover the left half of the housing 1 from both sides in the Y-axis. A front wall 810 of the other of the pair of pinching units 80 (referred to as a "second pinching unit 80") covers the substantially right half of the front surface of the housing 1. Three side walls 820 of the second pinching unit 80 cover the right side of the housing 1 and also cover the right half of the housing 1 from both sides in the Y-axis. As shown in FIG. 1, in the front side of the housing 1, the opening 800 is defined between the front walls 810 of the pair of pinching units 80. That is, the pair of pinching units 80 are arranged such that the opening 800 is formed between the pair of pinching units 80 on the front side of the housing 1. Accordingly, the skin introducing unit 8 has the opening 800.

The front surface of the front wall 810 of each pinching unit 80 has a contact surface 85 (see FIG. 1) configured to be in contact with the skin 92. According to the hair control device 10 of the present embodiment, the opening 800 is provided in the contact surface 85.

Each pinching unit 80 is supported by the housing 1 to be movable in the left-right directions along the X-axis (see the two-dot chain line in FIG. 1). When the pinching unit 80 moves along the X-axis, the dimension (opening width) Y1 measured along the X-axis of the opening 800 changes. That is, the pinching unit 80 is movable relative to the housing 1 along a moving axis A1 (X-axis) so as to change an opening size of the opening 800. The moving axis A1 intersects (substantially at right angles) with the opening direction of the opening 800 (the normal direction of the imaginal plane containing the opening 800; a direction along which the light of the light emitting unit 2 is emitted to the outside through the opening 800; Z-axis).

As shown in FIGS. 3, 4, the pair of pinching units 80 are coupled together with a hinge unit 89. The pair of pinching units 80 are coupled together with the hinge unit 89 such that the pair of pinching units 80 is configured to turn (pivot) around the hinge unit 89 (around the Y-axis) between a close position where tip ends 83 of the pinching units 80 are approached each other (see FIG. 3) and a separated position where the tip ends 83 of the pinching units 80 are distant from each other (see FIG. 4). For example1, when the user holds the left side surface and the right side surface of the device with one's hand and grips it, the pair of pinching units 80 transitions to the close position. When the user releases one's hand, the pair of pinching units 80 transitions to the separated position by the spring force of a spring or the like. According to the hair control device 10 of the present embodiment, the pair of pinching units 80 (the skin introducing unit 8) is not coupled to the housing 1. Specifically, the housing 1 is housed in a space surrounded by the pair of pinching units 80, whereby the skin introducing unit 8 is held by the housing 1. According to the hair control device 10 of the present embodiment, therefore, the skin introducing unit 8 is an attachment configured to be removably attached to the housing 1. In other words, at least part of the skin introducing unit 8 is detachably attached to the housing 1.

When the pinching unit 80 is moved in a direction to reduce the dimension Y1 of the opening 800 (moved from the two-dot chain line position to the solid line position in FIG. 1) in a state where the contact surface 85 of the pinching unit 80 is in contact with the surface of the skin 92, the skin 92 is pinched between the pair of pinching units 80 and deformed, and thus is drawn into the recessed space S1 (see FIG. 5). That is, the pinching units 80 are configured to pinch the skin 92 therebetween to introduce the skin 92 into the recessed space S1.

According to the embodiment, the dimension Y1 of the opening 800 measured along the moving axis A1 (X-axis) in a state where the pinching unit 80 is to be moved to a position where the size of the opening 800 is the smallest (i.e., in the close position) falls within the range equal to or greater than 0 mm and equal to or smaller than 30 mm. The dimension Y1 of the opening 800 measured along the moving axis A1 (X-axis) in the state where the size of the opening 800 is the smallest may preferably fall within the range equal to or greater than 2 mm and equal to or smaller than 30 mm. Since the depth of the hair matrix 912 in the skin 92 is equal to or greater than 1 mm even in the thinnest region of the skin 92, the device having the dimension Y1 of the opening 800 equal to or greater than 2 mm in the above state can facilitate sufficiently introducing the hair matrix 912 into the recessed space S1. Contrary, when the device has the dimension Y1 of the opening 800 greater than 30 mm in the above state, it may be difficult to introduce the skin 92 into the recessed space S1 sufficiently.

In a state where the pinching units 80 are located at the position exhibiting the maximum opening 800 size (i.e., at the separated position), the opening 800 may have a dimension measured along the moving axis A1 (X-axis) equal to or greater than the twice of the dimension D1 measured along the moving axis A1 (X-axis) of the opening plane of the recessed space S1. As shown in FIG. 4, in the state where the opening 800 has the maximum size (at the separated position), the dimension D2 between a tip end 83 of one pinching unit 80 and one end of the opening plane of the recessed space S1 measured along the moving axis A1 (X-axis) may be equal to or greater than the half of the dimension D1, for example. Moreover, in the state where the opening 800 has the maximum size (at the separated position), the dimension D3 between a tip end 83 of the other pinching unit 80 and the other end of the opening plane of the recessed space S1 measured along the moving axis A1 (X-axis) may be equal to or greater than the half of the dimension D1. In short, the following relation may be satisfied: D1+D2+D3≥2*D1. In this configuration, when the pair of pinching units 80 moves from the separated positions (see FIG. 4) to the close positions (see FIG. 3), the tip ends 83 of the pair of pinching units 80 totally move along the moving axis A1 by a distance equal to or greater than the dimension D1 described above. This can facilitate pinching the skin 92 and introducing the skin 92 into the recessed space S1 sufficiently.

Described this from another point of view, each pinching unit 80 includes a base part 81 and a protruding part 82, as shown in FIG. 1.

The base part 81 is a part of each pinching unit 80 and is supported by the housing 1 to be movable relative to the housing 1. According to the hair control device 10 of the present embodiment, the base part 81 includes a part (specifically, other than a part having a planar front surface along the X-axis) of the front wall 810, and three side walls 820. The housing 1 is housed in the space surrounded by the base parts 81 of the pair of pinching units 80, whereby the base parts 81 are supported by the housing 1 to be movable relative to the housing 1. As shown in FIG. 1, in the cross section perpendicular to the Y-axis and taken along a plane containing the light emitting unit 2 and the light transmission member 3, the base part 81 includes a substantially L-shaped (or reverse L-shaped) portion (i.e., a part of the front wall 810 and one side wall 820).

The protruding part 82 protrudes from the base part 81 along the moving axis A1. According to the hair control device 10 of the present embodiment, the tip end 83 of the protruding part 82 constituting one edge (an edge extending along the Y-axis) of the opening 800. According to the hair control device 10 of the present embodiment, the protruding part 82 protrudes along the moving axis A1 such that the tip end 83 of the protruding part 82 overlaps the light transmission piece 30 when viewed in the Z-axis in a state where the pinching units 80 are closed (see FIGS. 1, 3).

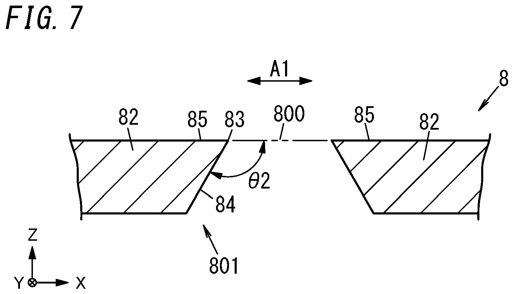

According to the hair control device 10 of the present embodiment, in an imaginal plane containing the moving axis A1 (i.e., X-axis) and an opening direction of the opening 800 (i.e., Z-axis), the protruding part 82 of the pinching unit 80 has an inclined surface shape including an inclined part 801, as shown in FIG. 7. That is, the protruding part 82 has an inclined surface 84 that crosses the moving axis A1 by an angle θ2, as shown in FIG. 7. According to the hair control device 10 of the present embodiment, the angle θ2 is an obtuse angle (i.e., greater than 90° and smaller than 180°). The angle θ2 is preferably equal to or smaller than 175°. When the angle θ2 is the obtuse angle, the tip end 83 of the protruding part 82 can dig into the skin 92 to facilitating the introduction of the skin 92 into the recessed space S1. That is, the function for introducing the skin 92 into the recessed space S1 can be improved.

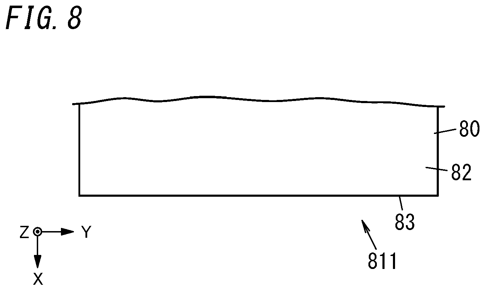

Moreover, according to the hair control device 10 of the present embodiment, when viewed in the opening direction of the opening 800 (i.e., when viewed in the Z-axis), the tip end 83 of the protruding part 82 of the pinching unit 80 has a straight line shape including a straight part 811, as shown in FIG. 8. When the tip end 83 of the protruding part 82 has the straight line shape, it can uniformly provide the force to the skin 92 in the Y-axis.

As described above, the hair control device 10 of the present embodiment includes the skin introducing unit 8 configured to introduce the skin 92 into the recessed space S1. This can facilitate the skin 92 to be brought into (close) contact with the light exit surface 13 and can uniquely set a positional relation between the light emitting unit 2 and the skin 92 even in a repeated use. This can efficiently provide the damage to the hair matrix 912 to improve the skin 92 treatment efficiency, for example.

### (1.3) Variations of Embodiment 1

Variations of the embodiment 1 will be described. In the following description, the embodiment 1 described above may be referred to as a "basic example (of the embodiment 1)". In the description of the variations, components that are substantially the same as those of a basic aspect may be appropriately omitted. The basic example and the variations may be readily modified depending on a design choice or any other factor, without departing from a true spirit and scope of the present disclosure.

### (1.3.1) First Variation

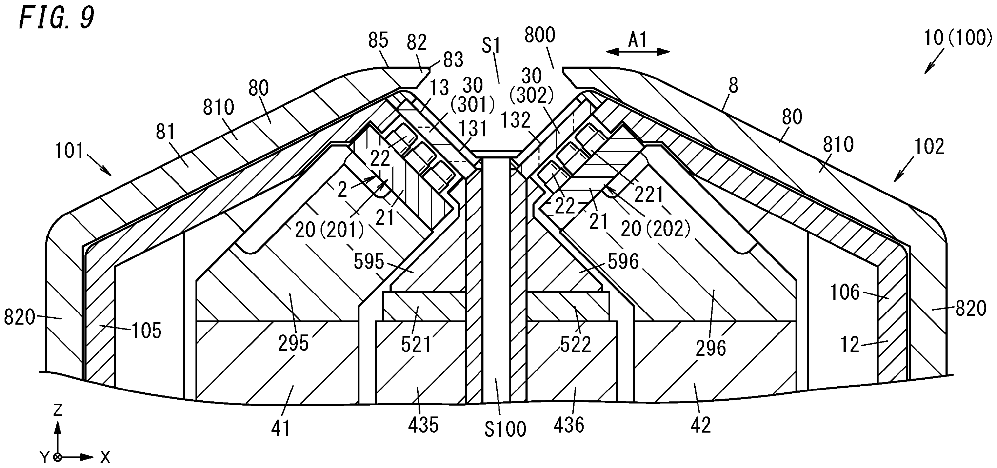

A hair control device 10 (photocosmetic device 100) of the present variation will be described with reference to FIG. 9. The hair control device 10 of the present variation differs from the hair control device 10 of the basic example in a configuration of a housing 1, a heat dissipation part 4, a cooler 5 etc.

As shown in FIG. 9, according to the hair control device 10 of the present variation, the housing 1 includes a first housing part 105 in which a first light source unit 201 is housed and a second housing part 106 in which a second light source unit 202 is housed. The first housing part 105 and the second housing part 106 are disposed separately (separated) from each other in a direction in which the first light source unit 201 and the second light source unit 202 are arranged.

According to the hair control device 10 of the present variation, the cooler 5 includes a first Peltier device 521 and a second Peltier device 522. The first Peltier device 521 is housed in the first housing part 105 and configured to cool a first light transmission piece 301. The second Peltier device 522 is housed in the second housing part 106 and configured to cool a second light transmission piece 302.

A first coupling member 595 is disposed on a front surface of the first Peltier device 521. The first coupling member 595 is in contact with the first light transmission piece 301. The front surface of the first Peltier device 521 is thermally connected to the first light transmission piece 301 via the first coupling member 595.

A second coupling member 596 is disposed on a front surface of the second Peltier device 522. The second coupling member 596 is in contact with the second light transmission piece 302. The front surface of the second Peltier device 522 is thermally connected to the second light transmission piece 302 via the second coupling member 596.

According to the hair control device 10 of the present variation, a third heat dissipation part 43 includes a first part 435 coupled to the first Peltier device 521 and a second part 436 coupled to the second Peltier device 522.

Specifically, the hair control device 10 of the present variation includes a first light source block 101 and a second light source block 102.

The first light source block 101 includes the first light source unit 201, the first light transmission piece 301, a first holding member 295, a first heat dissipation part 41, the first Peltier device 521, the first coupling member 595, the first part 435 of the third heat dissipation part 43, and the first housing part 105. The first housing part 105 has a box shape and houses therein the first light source unit 201, the first holding member 295, the first heat dissipation part 41, the first Peltier device 521, the first coupling member 595, and the first part 435 of the third heat dissipation part 43. The first housing part 105 holds the first light transmission piece 301 such that the first light transmission piece 301 is positioned on an optical axis of the first light source unit 201.

The second light source block 102 includes the second light source unit 202, the second light transmission piece 302, a second holding member 296, a second heat dissipation part 42, the second Peltier device 522, the second coupling member 596, the second part 436 of the third heat dissipation part 43, and the second housing part 106. The second housing part 106 has a box shape and houses therein the second light source unit 202, the second holding member 296, the second heat dissipation part 42, the second Peltier device 522, the second coupling member 596, and the second part 436 of the third heat dissipation part 43. The second housing part 106 holds the second light transmission piece 302 such that the second light transmission piece 302 is positioned on an optical axis of the second light source unit 202.

According to the hair control device 10 (the photocosmetic device 100) of the present variation, at least one of (in the variation, both of) the first light source block 101 or the second light source block 102 is movable in the direction (direction along the X-axis) in which the first light source unit 201 and the second light source unit 202 are arranged. That is, the first light source block 101 (the first housing part 105 in which the components are housed) and the second light source block 102 (the second housing part 106 in which the components are housed) are movable along an axis (X-axis) along which the pair of pinching units 80 are movable.

According to the hair control device 10 of the present variation, the first housing part 105 and the second housing part 106 are fixed to a third case 17 with a hinge mechanism and are turnable (pivotable) around the hinge mechanism (around the Y-axis). Accordingly, the first housing part 105 and the second housing part 106 are movable along the direction of the X-axis.

According to the hair control device 10 of the present variation, a gap (space S100) remains between the first housing part 105 and the second housing part 106 even in a state where the first housing part 105 and the second housing part 106 are approached closest each other. (see FIG. 9).

According to the hair control device 10 of the present variation, the first housing part 105 and the second housing part 106 are not fixed to the pair of pinching units 80, but are movable separately from the pair of pinching units 80. However, the movements of the first housing part 105 and the second housing part 106 are restricted some extent by the pair of pinching units 80. For example, when the pair of pinching units 80 are moved from the separated positions to the close positions, the first housing part 105 and the second housing part 106 are pushed by inner surfaces of the pinching units 80 to move in direction for approaching each other.

The hair control device 10 of the present variation can pinch the skin 92 between the first light source block 101 and the second light source block 102 by, e.g., firstly the light exit surface 13 being brought into contact with the skin 92 in a state where the first light source block 101 and the second light source block 102 are distant from each other and a distance therebetween are set to be relatively large, and then the first light source block 101 and/or the second light source block 102 is moved to minimize the distance between the first light source block 101 and the second light source block 102. This allows the skin 92 to be further closely in contact with the light exit surface 13. Moreover, by moving the pair of pinching units 80 to directions of approaching each other, the skin 92 can be further introduced into the recessed space S1 by the pair of pinching units 80.

The hair control device 10 of the present variation may include a stopper mechanism configured to form a gap between the first housing part 105 and the second housing part 106 even when the first light source block 101 and the second light source block 102 are approached closest each other. That is, the hair control device 10 may further include the stopper mechanism configured to restrict the movement of at least one of the first housing part 105 or the second housing part 106 such that the gap remains between the first housing part 105 and the second housing part 106 even in a state where the first housing part 105 and the second housing part 106 are approached closest each other. The stopper mechanism may include a protrusion protruding from one of the first housing part 105 and the second housing part 106 toward the other. The stopper mechanism may include a spring provided in the hinge mechanism and configured to give an elastic force to move the first housing part 105 and the second housing part 106 away from each other. The stopper mechanism may include a structure that restricts a movable range of a sliding mechanism. The stopper mechanism can prevent the skin 92 from being tightly caught between the first housing part 105 and the second housing part 106. The uncomfortable feeling to be caused in a user can be reduced. However, this should not be construed as limiting. Alternatively, the hair control device 10 may be configured such that the first housing part 105 and the second housing part 106 come in contact with each other without gap therebetween when the first light source block 101 and the second light source block 102 are approached closest each other.

According to the present variation, the light exit surface 13 can be in contact with the skin 92 more closely, which can improve the treatment efficiency for treating the skin 92.

### (1.3.2) Second Variation

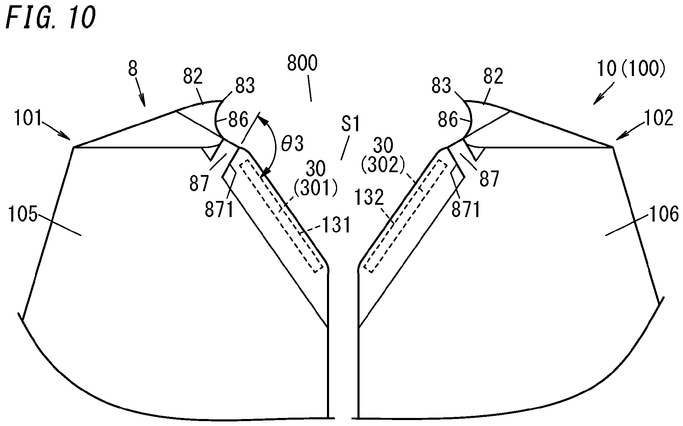

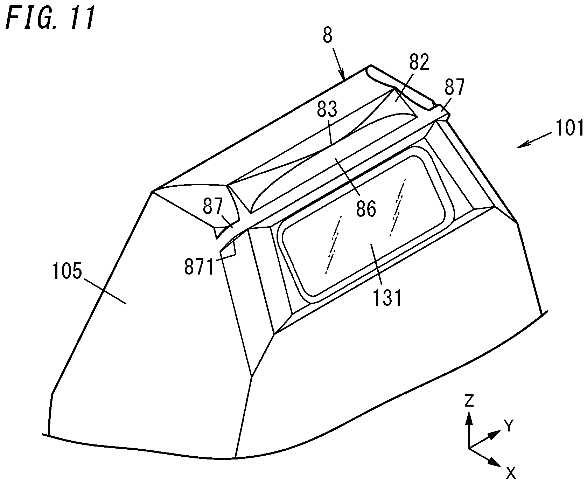

A hair control device 10 (photocosmetic device 100) of the present variation will be described with reference to FIGS. 10, 11. The hair control device 10 of the present variation differs from the hair control device 10 of the first variation in a configuration of a housing 1, a skin introducing unit 8 etc.

As shown in FIGS. 10, 11, according to the hair control device 10 of the present variation, the skin introducing unit 8 (pinching unit 80) is integrated with the housing 1. Specifically, one of a pair of pinching units 80 is integrated with a first housing part 105, and the other of the pair of pinching units 80 is integrated with a second housing part 106.

When the skin introducing unit 8 is integrated with the housing 1, it is possible to prevent the occurrence of the situation where the skin 92 is bitten between the skin introducing unit 8 and the housing 1. This can reduce the uncomfortable feeling to be caused in a user.

As shown in FIGS. 10, 11, a protruding part 82, which is a part of the pinching unit 80 and faces a recessed space S1, has a concave shaped recessed to be apart from the recessed space S1 when viewed in the Y-axis. That is, the protruding part 82 has a recessed surface 86 recessed to be apart from the recessed space S1. When the first housing part 105 and the second housing part 106 are moved to approach each other, the skin 92 will be introduced into a space inside the recessed surface 86 and come into contact with the recessed surface 86, for example. In this state, the tip end 83 of the protruding part 82 (specifically, an edge portion of the recessed surface 86) bites into a surface of the skin 92, so that the pinching unit 80 is less likely to be slipped over the skin 92. This can prevent the skin 92 introduced into the space inside the recessed surface 86 from being fallen out from the space. As a result, the skin 92 inside the recessed space S1 is also less likely to be moved (slipped), which can prevent the skin 92 from escaping (slipping out) from the recessed space S1.

The skin introducing unit 8 further includes an assisting part (first assisting part) 87. The first assisting part 87 has a plate shape. The first assisting part 87 has a contact surface 871 configured to, when a skin 92 is introduced into the recessed space S1, come into contact with a part around the skin 92 introduced into the recessed space S1. In the variation, the recessed space S1 has a groove shape extending in one direction (a direction of the Y-axis). The first assisting part 87 protrudes from the housing 1 (a first housing part 105 or a second housing part 106) in a direction along which the recessed space S1 extends (i.e., along the direction of the Y-axis). The contact surface 871 and a light exit surface 13 (a first inclined surface 131 or a second inclined surface 132) forms an angle θ3 falling within the range from 90° to 150°, for example.

The skin introducing unit 8 having the first assisting part 87 can prevent the skin 92 from escaping (slipping out) from the recessed space S1.

### (1.3.3) Third Variation

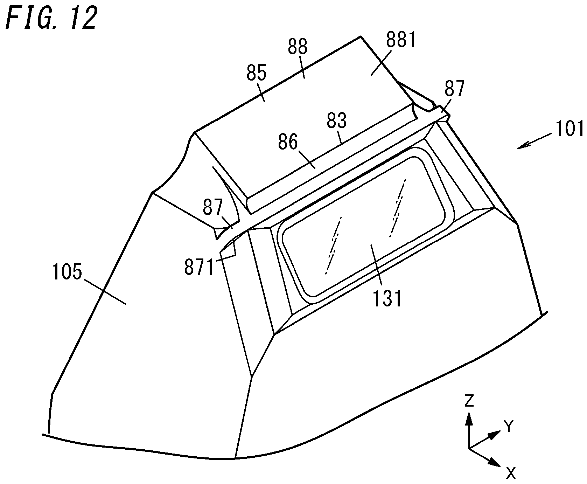

A hair control device 10 (photocosmetic device 100) of the present variation will be described with reference to FIG. 12. The hair control device 10 of the present variation differs from the hair control device 10 of the second variation in a configuration of a skin introducing unit 8 etc.

As shown in FIG. 12, the skin introducing unit 8 further includes an assisting part (second assisting part) 88. The second assisting part 88 has a flare shape. The second assisting part 88 is provided on an opposite side of a light exit surface 13 (a first inclined surface 131 or a second inclined surface 132) with respect to a tip end 83 of a protruding part 82. The second assisting part 88 is configured to, when the skin 92 is introduced into the recessed space S1, come into contact with a part around the skin 92 introduced into the recessed space S1.

According to the hair control device 10 of the present variation, the second assisting part 88 has an inclined surface 881. The inclined surface 881 of the second assisting part 88 and the light exit surface 13 (the first inclined surface 131 or the second inclined surface 132) forms an angle (angle in the X-Z plane) falling within the range from 120° to 180°, for example. The inclined surface 881 of the second assisting part 88 and the contact surface 871 of the assisting part 87 forms an angle (angle in the X-Z plane) of about 90°, for example.

The hair control device 10 of the present variation can further prevent the skin 92 from escaping (slipping out) from the recessed space S1 by virtue of the frictional force between the skin 92 and the second assisting part 88.

### (1.3.4) Fourth Variation

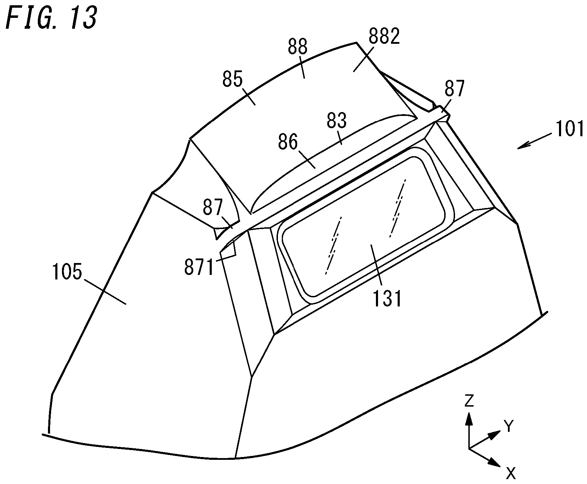

A hair control device 10 (photocosmetic device 100) of the present variation will be described with reference to FIG. 13. The hair control device 10 of the present variation differs from the hair control device 10 of the third variation in a configuration of a second assisting part 88 etc.

According to the hair control device 10 of the present variation, the second assisting part 88 has a curved surface 882 having an arch-like shape. The curved surface 882 is curved such that both ends thereof in a direction of the Y-axis protrudes compared to the center thereof.

When the second assisting part 88 has the curved surface 882, the pressure applied from the second assisting part 88 to the skin 92 is greater in the center than that in the both ends in the direction of the Y-axis. Accordingly, the pushing amount of the skin 92 by the second assisting part 88 can be uniformized in the Y-axis.

### (1.3.5) Other Variations

In one variation, a pair of pinching units 80 may be coupled to a housing 1 with a coupling structure such as a hinge.

In one variation, both of a pair of pinching units 80 may be movable relative to a housing 1, or either one of a pair of pinching units 80 may be movable relative to a housing 1. Either one of the pair of pinching units 80 may be fixed so as not to move relative to the housing 1. In this case, the other of the pair of pinching units 80 may be movably coupled to the housing 1 or may be movably coupled to the one (counter) pinching unit 80. At least part of the skin introducing unit 8 may be continuously integrated with the housing 1.

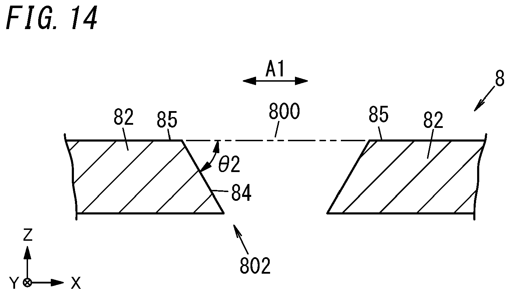

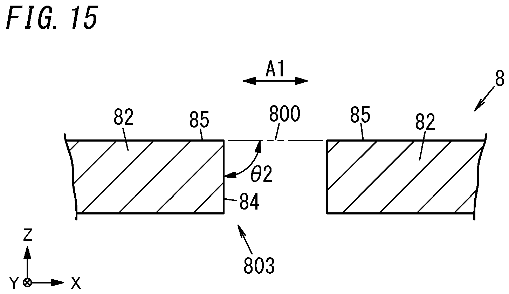

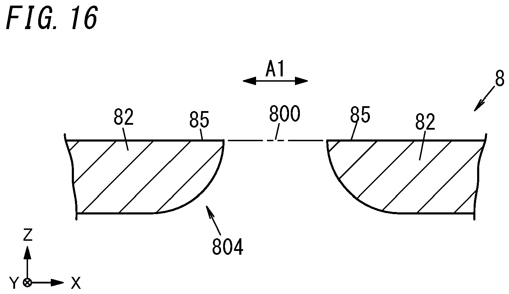

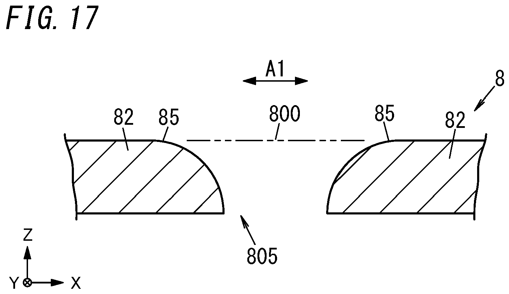

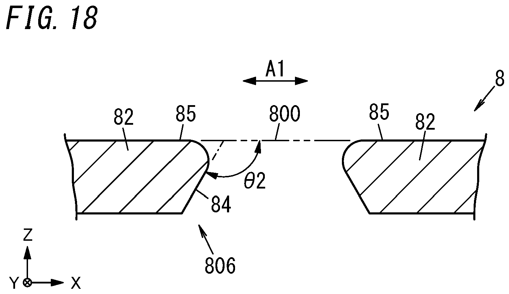

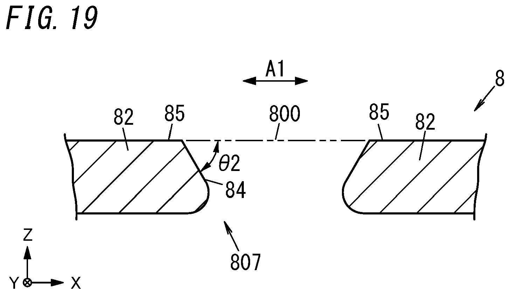

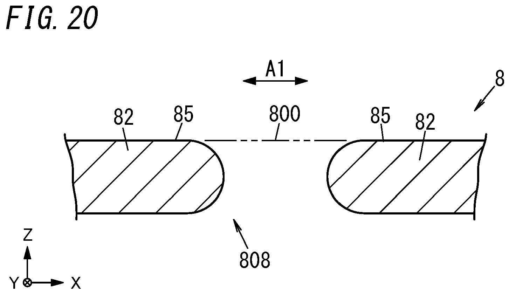

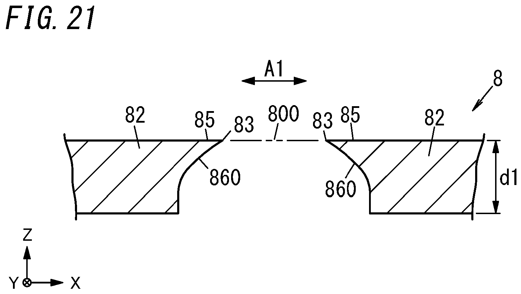

A protruding part 82 of a pinching unit 80 is not limited to have an inclined surface shape (see FIG. 7) having the inclined surface 84 (inclined part 801) that crosses the moving axis A1 by the angle θ2 of an obtuse angle, in the imaginal plane containing the moving axis (X-axis) and the opening direction of the opening 800 (direction of the Z-axis). In one alternative (first alternative), a protruding part 82 of a pinching unit 80 has, in an imaginal plane containing a moving axis A1 and an opening direction of an opening 800, an inclined surface shape having an inclined surface 84 (inclined part 802) that crosses a moving axis A1 by an angle θ2 of an acute angle, as shown in FIG. 14. In this case, the angle θ2 may preferably be equal to or greater than 15°. The protruding part 82 exhibiting the angle θ2 of the acute angle can introduce the skin 92 into the recessed space S1 in a state where the shape of the skin 92 follows the inclined surface 84. In one alternative (second alternative), a protruding part 82 of a pinching unit 80 has, in an imaginal plane containing a moving axis A1 and an opening direction of an opening 800, a square bracket shape having a wall part 803 that crosses a moving axis A1 by an angle θ2 of right angles, as shown in FIG. 15. In one alternative (a third alternative, or a fourth alternative), a protruding part 82 of a pinching unit 80 has, in an imaginal plane containing a moving axis A1 and an opening direction of an opening 800, a quarter of a circle shape including a circular arc part 804, 805, as shown in FIGS. 16, 17. In one alternative (a fifth alternative or a sixth alternative), a protruding part 82 of a pinching unit 80 has, in an imaginal plane containing a moving axis A1 and an opening direction of an opening 800, an inclined surface shape including a chamfered inclined part 806, 807, as shown in FIGS. 18, 19. In one alternative (seventh alternative), a protruding part 82 of a pinching unit 80 has, in an imaginal plane containing a moving axis A1 and an opening direction of an opening 800, a semicircular shape including a semicircular part 808, as shown in FIG. 20. In one alternative (seventh alternative), a protruding part 82 of a pinching unit 80 has, in an imaginal plane containing a moving axis A1 and an opening direction of an opening 800, a recessed surface 860 recessed from a tip end 83 toward a base part of the protruding part 82, as shown in FIG. 21. In this case, the recessed surface 860 may be a curved surface. The radius of curvature R1 of the recessed surface 860 may satisfy the relation d1/2≤R1≤2*d1, where "d1" denotes a dimension of the protruding part 82 measured along the Z-axis direction. In this case, the angle of the tip end 83 of the protruding part 82 may be equal to or smaller than 45°. In one alternative, a protruding part 82 of a pinching unit 80 has, in an imaginal plane containing a moving axis A1 and an opening direction of an opening 800, an arc shape including an arc part. In one alternative, a protruding part 82 of a pinching unit 80 has, in an imaginal plane containing a moving axis A1 and an opening direction of an opening 800, a curved surface shape including two or more curves having different curvatures. In short, in the imaginal plane containing the moving axis A1 and the opening direction of the opening 800, the protruding part 82 of the pinching unit 80 may have at least one shape selected from a group consisting of a square bracket shape, an inclined surface shape, a semicircular shape, a quarter of a circle shape, an arc shape, and a curved surface shape including two or more curves having different curvatures. When the protruding part 82 has the shape to easily grab the skin 92, the skin 92 can be easily introduced into the recessed space S1. Moreover, when the protruding part 82 has the shape including a curved surface, the stimulation on the skin 92 can be reduced.

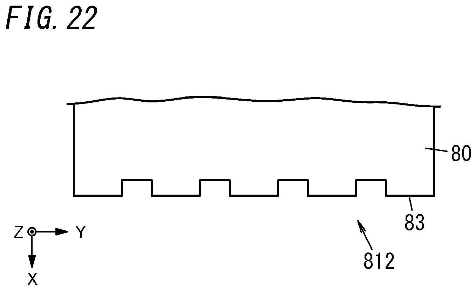

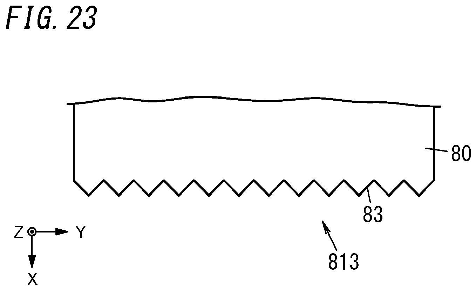

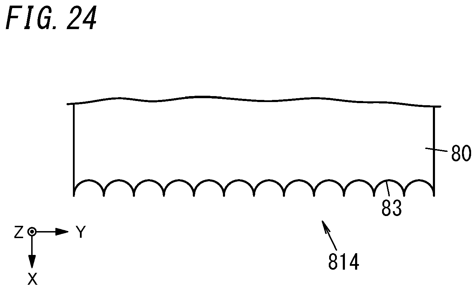

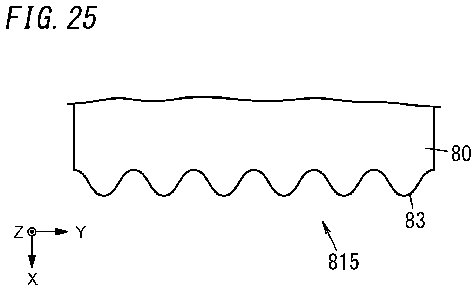

A tip end 83 of a protruding part 82 of a pinching unit 80 is not limited to have a straight line shape (see FIG. 8) when viewed in the opening direction of the opening 800 (when viewed in the Z-axis). In one alternative (ninth alternative), a tip end 83 of a protruding part 82 of a pinching unit 80 has, when viewed in an opening direction of an opening 800, a comb shape including a comb part 812, as shown in FIG. 22. In one alternative (tenth alternative), a tip end 83 of a protruding part 82 of a pinching unit 80 has, when viewed in an opening direction of an opening 800, a triangle wave shape including a triangle wave part 813, as shown in FIG. 23. In one alternative (eleventh alternative), a tip end 83 of a protruding part 82 of a pinching unit 80 has, when viewed in an opening direction of an opening 800, a reverse semicircular wave shape including a reverse semicircular wave part 814, as shown in FIG. 24. In one alternative (twelfth alternative), a tip end 83 of a protruding part 82 of a pinching unit 80 has, when viewed in an opening direction of an opening 800, a circular wave shape including a circular wave part 815, as shown in FIG. 25. In short, when viewed in the opening direction of the opening 800, the tip end 83 of the protruding part 82 of the pinching unit 80 may have at least one shape selected from a group consisting of a straight line shape, a comb shape, a triangle wave shape, a reverse semicircular wave shape, and a circular wave shape. When the tip end 83 of the protruding part 82 has the shape to easily grab the skin 92, the skin 92 can be easily introduced into the recessed space S1. Moreover, when the tip end 83 of the protruding part 82 has the shape including a curved surface, the stimulation on the skin 92 can be reduced.

In one variation, a skin introducing unit 8 is not limited to include the pinching unit 80. Alternatively, a skin introducing unit 8 may have a structure including a roller to introduce the skin 92 into the recessed space S1, for example.

In one variation, in a state where the pinching units 80 are closed (at the close positions), a tip end 83 of a protruding part 82 of a pinching unit 80 may be located on an outside of a light transmission piece 30 in the X-axis. Specifically, when a left pinching unit 80 is located at the most right position, a tip end 83 of a protruding part 82 of the left pinching unit 80 may be located on the left of a left edge of a left light transmission piece 30 (first light transmission piece 301). Moreover, when a right pinching unit 80 is located at the most left position, a tip end 83 of a protruding part 82 of the right pinching unit 80 may be located on the right of a right edge of a right light transmission piece 30 (second light transmission piece 301).

In one variation, a housing 1 does not have to house therein an entire of a light emitting unit 2. A housing 1 may house a light emitting unit 2 with a part of a light emitting unit 2 (e.g., a part of a substrate 21) protruding from the housing 1, for example.

In one variation, a substrate 21 and a light transmission member 3 (a first light transmission piece 301 or a second light transmission piece 302) may not have to be in parallel to each other, but may be inclined. In this case, an inclined angle between a light transmission member 3 (light transmission piece 30) and a substrate 21 may be set in consideration with a loss of light caused by the total refection. The inclined angle between the light transmission piece 30 and the substrate 21 may be set such that (majority) of the light emitted from the light source 22 and traveling in the light transmission piece 30 enters a light exit surface 13 (front surface of the light transmission piece 30) by an incident angle equal to or smaller than the critical angle. The critical angle means the smallest angle of incidence that yields total reflection. The critical angle depends on the refractive index of the material of the light transmission piece 30 and the refractive index of the air. Disposing the substrate 21 and the light transmission member 3 in this manner can reduce the loss of light caused by the total reflection, and makes it easy to illuminate the desired amount of the light on the skin 92.

In one variation, a light source unit 20 may include an optical member configured to change a direction of an optical path of light of a light source 22 by the reflection or the refraction, such as a mirror, a half mirror, or a prism. In this case, an optical axis of the light source unit 20 may not coincide with the optical axis of the light source 22.

In one variation, a light transmission member 3 (light transmission piece 30) may have a shape serving as a lens.

In one variation, a photocosmetic device 100 may not include a light transmission member 3.

In one variation, the number of light source units 20 included in a light emitting unit 2 is not limited to two, but may be three or more.

In one variation, a light exit surface 13 does not have to have a V-groove shape when viewed in the Y-axis, but may have a recessed groove shape when viewed in the Y-axis for example. In this case, a light transmission member 3 (light transmission piece 30) may be disposed in a bottom of the recessed groove, and a light source unit 20 may be disposed to face the light transmission piece 30. The light exit surface 13 may include a first inclined surface 131 and a second inclined surface 132, which extend from both ends of the bottom of the recessed groove at right angles, and which face each other.

In one variation, a pinching unit 80 (specifically, a protruding part 82) may be made from rubber. This can increase the friction force between the skin 92 and the pinching unit 80 to prevent the slipping of the skin 92. Moreover, the uncomfortable feeling to be caused in a user can be reduced. The pinching unit 80 may be made from resin, hard resin, or soft resin.

### (2) Embodiment 2

### (2.1) Overview of Embodiment 2

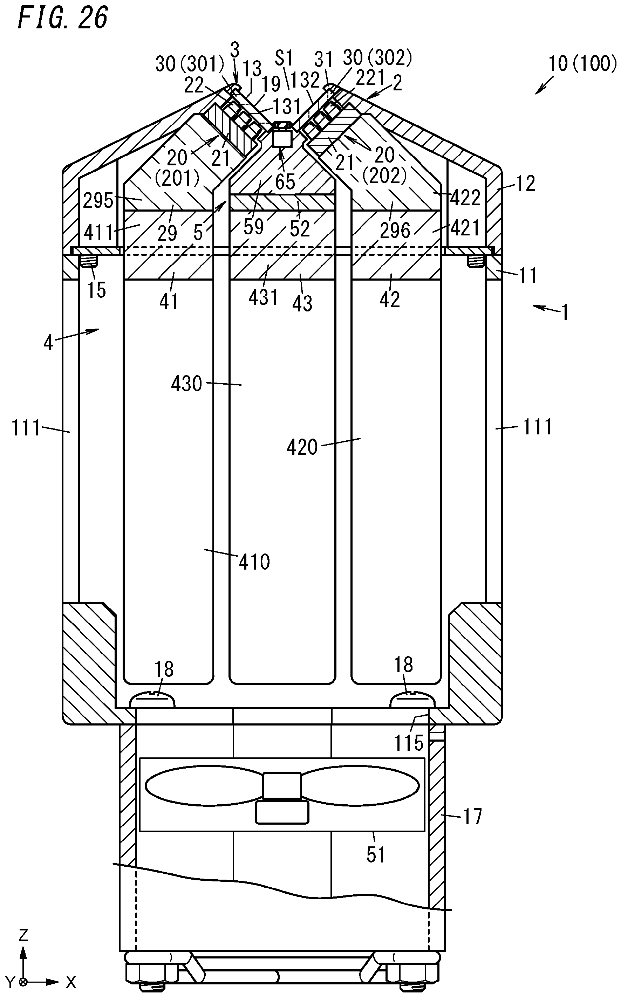

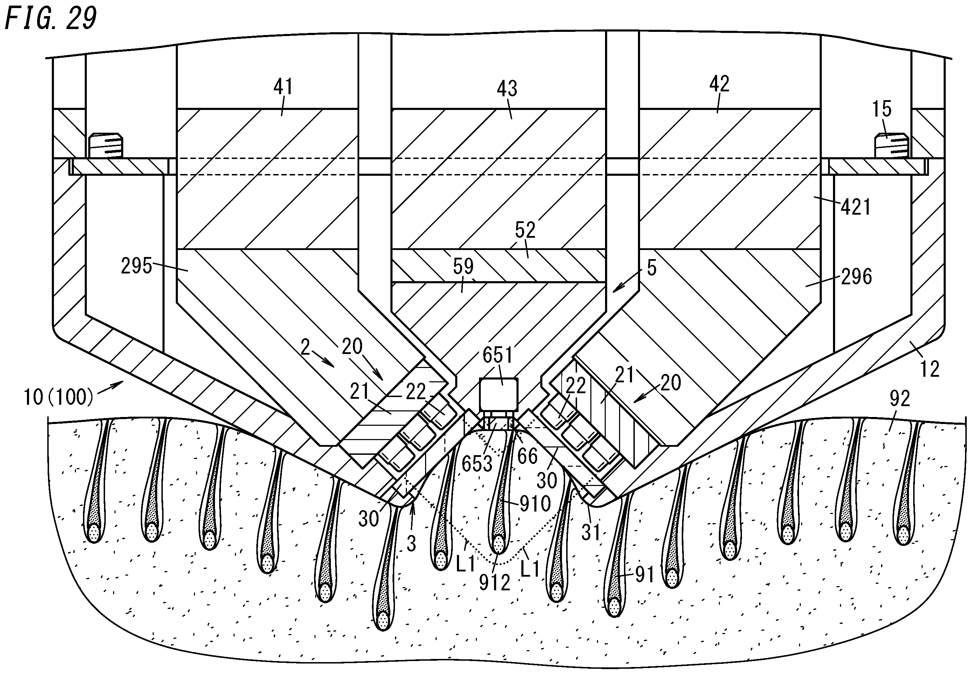

A photocosmetic device 100 of the present embodiment (see FIGS. 26 to 30) is a device for illuminating light to a skin 92 (see FIG. 29) to cause absorption of the light in the skin 92 to treat the skin 92 with the function of the absorbed light.

JP2020-39894A (hereinafter, referred to as a "reference document") discloses a skin treatment system. The skin treatment system includes a treatment device and a camera. The treatment device is configured and arranged such that, in an operational state of the skin treatment system, the treatment device treats a treatment region of a mammal body by physically acting upon the mammal body in said treatment region. The camera is configured and arranged such that, in the operational state of the skin treatment system, the camera captures at least one image of skin of the mammal body having an imaging region and provides captured image data representative of said at least one captured image. Said imaging region has a predefined spatial relationship with said treatment region.

The reference document recites, as examples of the predefined spatial relationship, coinciding, partly overlapping and disjointing the imaging region and the treatment region.

The reference document is silent on the positional relation of the camera with respect to the treatment device in the skin treatment system. The spatial relationship between the imaging region of the camera and the treatment region thus cannot be guaranteed. Therefore, the imaging region may be unintendedly diverged from the treatment region. This may reduce the treatment efficiency to treat the skin.

In order for the efficient treatment (e.g., facilitation of falling off of the hair 91 for hair control or hair removal) of the skin 92 with the light irradiation, it is desirable that sufficient amount of light of the photocosmetic device 100 reaches a part in the skin 92 desired to absorb the light (a part around the hair matrix 912 of the hair 910 to be removed; see FIG. 29). For this purpose, it is preferable that the ray of the light of the photocosmetic device 100 strikes a desired region (e.g., a region with the hair 910 to be removed) of the surface of the skin 92.

In this regard, the photocosmetic device 100 of the present embodiment includes a light emitting unit 2 configured to emit light, a housing 1 in which the light emitting unit 2 is housed, and an imaging unit 65, as shown in FIG. 26.

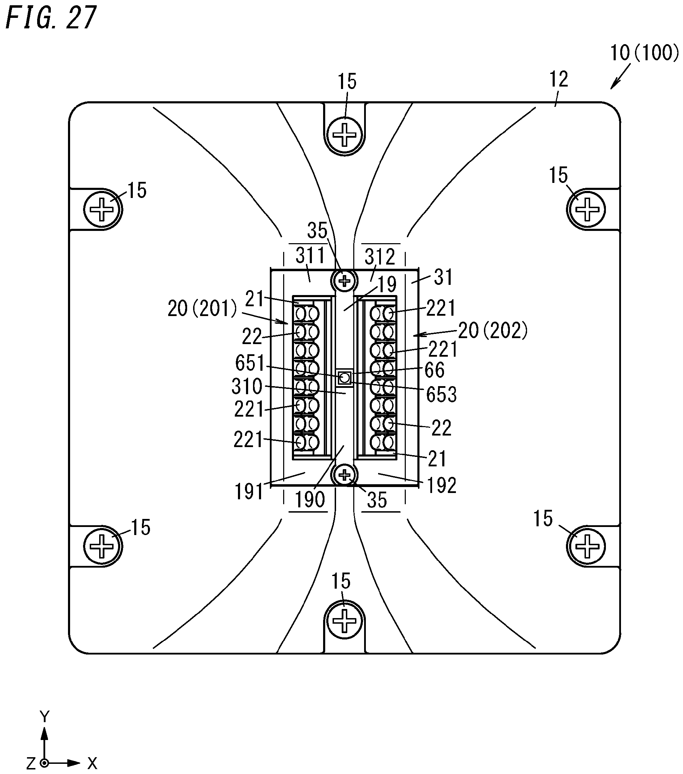

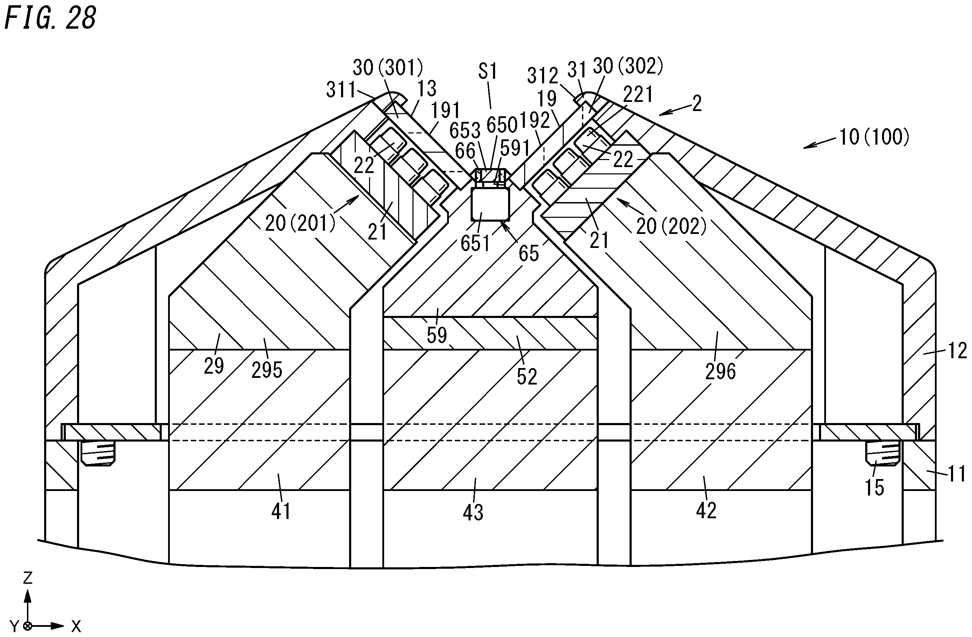

The housing 1 has a recess 19. The recess 19 of the housing 1 includes a bottom surface 190 (see FIG. 27), ant at least one (in the embodiment, a pair of) side surface(s) 191, 192 (see FIGS. 27, 28).

According to the photocosmetic device 100, the light of the light emitting unit 2 is emerged outside from the side surfaces 191, 192 of the recess 19. Moreover, according to the photocosmetic device 100, the imaging unit 65 has a light incident surface 650 in the bottom surface 190 of the recess 19 of the housing 1 and is configured to take an image based on the light entering through the light incident surface 650. The imaging unit 65 is housed in the housing 1 such that a space inside the recess 19 (i.e., a recessed space S1) is contained in a viewing angle of the imaging unit 65, for example. Accordingly, when an object (such as a surface of the skin 92) is present in the recessed space S1, the imaging unit 65 can take an image of the object. Moreover, the imaging unit 65 can take an image of an object present in front of the recessed space S1, that is, the imaging unit 65 can take an image of an object which is to be introduced into the recessed space S1.

According to the photocosmetic device 100 of the present embodiment, the light of the light emitting unit 2 is emerged outside from the side surfaces 191, 192 of the recess 19, and an image is taken by the imaging unit 65 having the light incident surface 650 located in the bottom surface 190 of the recess 19. Therefore, the imaging unit 65 can take an image of an area to which the light of the light emitting unit 2 is currently illuminated or is to be illuminated. Accordingly, an image taken region of the imaging unit 65 and a region to which the light of the light emitting unit 2 is illuminated surely coincide with each other with high reliability. This allows the light of the light emitting unit 2 to be illuminated on a desired region of the surface of the skin 92 with a high reliability, as a result of which the decline in the treatment efficiency to treat the skin 92 can be reduced.

### (2.2) Details of Embodiment 2

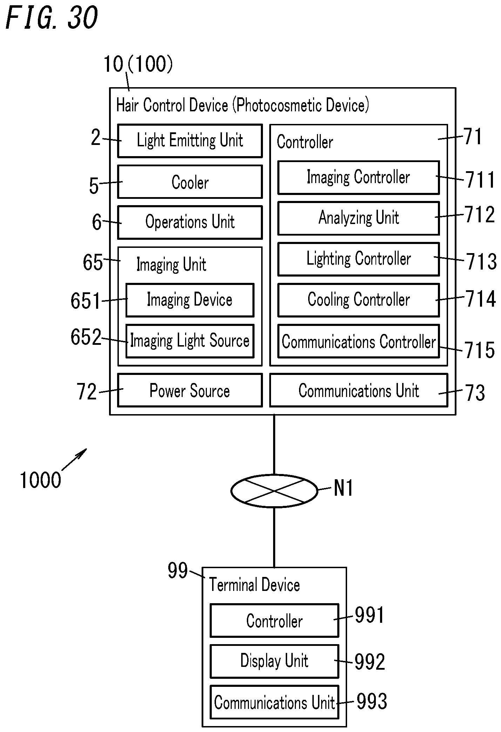

A photocosmetic system 1000 including the photocosmetic device 100 of the present embodiment is described below with reference to FIGS. 26 to 30. As shown in FIG. 30, the photocosmetic system 1000 includes the photocosmetic device 100 and a terminal device 99.

### (2.2.1) Photocosmetic Device

The photocosmetic device 100 (hair control device 10) of the present embodiment is described with reference to attached drawings.

As shown in FIGS. 26, 28, 30, the hair control device 10 includes the housing 1, a light exit surface 13, the light emitting unit 2, a light transmission member 3, a heat dissipation part 4, a cooler 5, an operations unit 6, the imaging unit 65, a thermal insulation part 66, a controller 71, a power source 72, and a communications unit 73. The heat dissipation part 4, the operations unit 6, and the power source 72 have substantially same configurations as those of the hair control device 10 of the embodiment 1 and thus the explanations thereof will be omitted.

The housing 1 houses therein the light emitting unit 2, the light transmission member 3, the heat dissipation part 4, the cooler 5, the imaging unit 65, the thermal insulation part 66, the controller 71, the power source 72, and the communications unit 73. The operations unit 6 is provided to an outer peripheral surface of the housing 1, for example.

As shown in FIG. 26, the housing 1 includes a first case 11, a second case 12, and a third case 17.

The first case 11 has a rectangular tube shape opened forward. The first case 11 includes left and right side walls in which a plurality of air vents 111 are formed to penetrate the side wall along the X-axis. The air vent 111 has a rectangular slot shape extending in the Z-axis. The first case 11 has an opening 115 in a rear surface. The first case 11 is a part to be held by the user when the hair control device 10 is used, for example.

The second case 12 is disposed on the front side of the first case 11 to cover the front opening of the first case 11. The second case 12 is coupled to the first case 11 with screws 15.

The third case 17 has a rectangular tube shape opened forward and rearward. The third case 17 is a bit smaller than the first case 11 when viewed in the Z-axis. The third case 17 is disposed on the rear side of the first case 11 such that a front opening of the third case 17 is continuous with the rear opening 115 of the first case 11. The third case 17 and the first case 11 are coupled together with screws 18.

As shown in FIGS. 26 to 28, a front surface of the housing 1 (i.e., a front surface of the second case 12) has the recess 19 recessed rearward. As shown in FIG. 27, the recess 19 includes the bottom surface 190 and the pair of side surfaces 191, 192. A normal direction of the bottom surface 190 of the recess 19 is along the Z-axis. The pair of side surfaces 191, 192 of the recess 19 extends diagonally forward from both ends in the X-axis of the bottom surface 190. The side surface 191 of the recess 19 extends diagonally left forward from the left edge (an edge on the negative side in the X-axis) of the bottom surface 190. The side surface 191 of the recess 19 is inclined relative to the bottom surface 190 by an angle of 135° with reference to a direction of the X-axis. The side surface 192 of the recess 19 extends diagonally right forward from the right edge (an edge on the positive side in the X-axis) of the bottom surface 190. The side surface 192 of the recess 19 is inclined relative to the bottom surface 190 by an angle of 45° with reference to the direction of the X-axis.

As shown in FIG. 27, the recess 19 extends along the Y-axis. The recess 19 of the housing 1 has a recessed groove shape. Specifically, the recess 19 has a recessed groove shape extending along an axis (Y-axis) crossing a front-rear axis (Z-axis).

The hair control device 10 has the light exit surface 13 (light exit part). According to the hair control device 10 of the present embodiment, the light exit surface 13 is located in the recess 19 in a front surface of the housing 1. The light exit surface 13 is located in the side surfaces 191, 192 of the recess 19. According to the hair control device 10 of the present embodiment, parts of the pair of side surfaces 191, 192 of the recess 19 in the front surface of the housing 1 constitutes the light exit surface 13.

The light exit surface 13 includes a first inclined surface 131 and a second inclined surface 132. A part of the side surface 191 of the recess 19 constitutes the first inclined surface 131. A part of the side surface 192 of the recess 19 constitutes the second inclined surface 132. The first inclined surface 131 and the second inclined surface 132 face each other in the X-axis.

As shown in FIG. 26, the recessed space S1 is defined as a space surrounded by the recess 19 (between the bottom surface 190, and the pair of side surfaces 191, 192). As shown in FIG. 29, the recessed space S1 is a space into which the skin 92 is introduced when the hair control device 10 is used. The recessed space S1 is also a space to which the light of the light emitting unit 2 is illuminated when the hair control device 10 is used, since the pair of side surfaces 191, 192 of the recess 19 defining the recessed space S1 partially constitutes the light exit surface 13 (the first inclined surface 131 and the second inclined surface 132).

As shown in FIG. 26, the light emitting unit 2 includes a plurality of (in the embodiment, two) light source units 20 (a first light source unit 201 and second light source unit 202) and a holding member 29.

As shown in FIG. 26, each of the plurality of light source units 20 includes a substrate 21 and a light source 22.

The substrate 21 has a rectangular plate shape. The substrate 21 extends along the Y-axis.

The light source 22 is mounted on the substrate 21. The light source 22 is mounted on a surface on the front side of the substrate 21.

As shown in FIGS. 26, 27, the light source 22 includes a plurality of LEDs 221. The light emitting unit 2 includes a plurality of LEDs.

The holding member 29 holds the substrates 21 of the plurality of (two) light source units 20. The holding member 29 includes a first holding member 295 holding the first light source unit 201 and a second holding member 296 holding the second light source unit 202. The plurality of light source units 20 (the first light source unit 201 and the second light source unit 202) are held by the holding member 29 such that optical axes of the plurality of light source units 20 cross each other on an outside of the housing 1.

As shown in FIG. 29, according to the hair control device 10 of the present embodiment, the plurality of light source units 20 (the first light source unit 201 and the second light source unit 202) are arranged such that rays of the light L1 of the plurality of light source units 20 cross each other in a region around the hair matrix 912 (bulb) in the skin 92 in a state where a surface of the skin 92 is in contact with the light exit surface 13 (in a state where the skin 92 is introduced into the recessed space S1).

As shown in FIG. 26, the light transmission member 3 includes a plurality of (two) light transmission pieces 30 (a first light transmission piece 301 and a second light transmission piece 302). The plurality of light transmission pieces 30 are arranged to correspond (one-to-one) to the plurality of light source units 20.

A front surface of the first light transmission piece 301 serves as the first inclined surface 131 of the light exit surface 13, and a front surface of the second light transmission piece 302 serves as the second inclined surface 132 of the light exit surface 13. Moreover, the front surface of the first light transmission piece 301 constitute a part of the side surface 191 of the recess 19, and the front surface of the second light transmission piece 302 constitute a part of the side surface 192 of the recess 19.

According to the hair control device 10 of the present embodiment, the recessed space S1 has a depth falling within the range greater than 0 mm and equal to or smaller than 30 mm. According to the present disclosure, the feature "depth of the recessed space S1" indicates a depth of the surface of the housing 1 from which the light is actually emitted. In one specific example, the "depth of the recessed space S1" is a dimension of the first inclined surface 131 (or the second inclined surface 132) measured along the Z-axis. The depth of the recessed space S1 may preferably fall within the range equal to or greater than 2 mm and equal to or smaller than 30 mm.

The recessed space S1 having the depth X1 equal to or greater than the lower limit of the above range allows the skin 92 to be sufficiently introduced into the recessed space S1, which can reduce the treatment time.

The light transmission member 3 is held by a holder 31. According to the hair control device 10 of the present embodiment, the holder 31 has a recessed plate shape when viewed laterally, and has openings into which the first light transmission piece 301 and the second light transmission piece 302 are respectively fitted.

Specifically, the holder 31 has a bottom wall 310 and a pair of side walls 311, 312, as shown in FIGS. 27, 28.

As shown in FIG. 27, the bottom wall 310 has a plate shape extending along the Y-axis. A normal direction of a front surface of the bottom wall 310 is along the Z-axis. A rectangular through hole is formed in the center in the Y-axis of the bottom wall 310. The front surface of the bottom wall 310 constitutes at least part of the bottom surface 190 of the recess 19.

The pair of side walls 311, 312 have plate shapes extending diagonally forward from left and right edges of the bottom wall 310.

The side wall 311 extends diagonally left forward from the left edge of the bottom wall 310. The side wall 311 is formed with the opening into which the first light transmission piece 301 is fitted. The front surface of the side wall 311 constitutes at least part of the side surface 191 of the recess 19.

The side wall 312 extends diagonally right forward from the right edge of the bottom wall 310. The side wall 312 is formed with the opening into which the second light transmission piece 302 is fitted. The front surface of the side wall 312 constitutes at least part of the side surface 192 of the recess 19.

The holder 31 is fixed to a coupling member 59 (described later) with screws 35 (see FIG. 27).

The cooler 5 is disposed to cool the light transmission member 3 (plurality of light transmission pieces 30). According to the present embodiment, the cooler 5 includes a Peltier device 52. The coupling member 59 is disposed on a front surface of the Peltier device 52.

A housing recess 591 (see FIG. 28) is formed in the center of the coupling member 59. The housing recess 591 has a cuboid shape and extends from a front surface of the coupling member 59 along the Z-axis.

As shown in FIG. 26, the cooler 5 further includes a cooling fan 51.

The imaging unit 65 includes: an imaging device 651; and at least one of an imaging light source 652 (see FIG. 30) or a light guide 653. According to the hair control device 10 of the present embodiment, the imaging unit 65 includes the imaging device 651 and the light guide 653. The imaging unit 65 further includes the imaging light source 652.

As shown in FIGS. 27, 28, the light guide 653 has a cuboid shape having a height along the Z-axis. The light guide 653 is a glass plate, for example. The height (dimension measured along the Z-axis) of the light guide 653 falls within the range equal to or greater than 0.1 mm and equal to or smaller than 10 mm, for example. When the light guide 653 is made of the glass plate and has an excess height, the imaging unit 65 tends to have a narrow viewing angle. Due to this reason, the height (dimension measured along the Z-axis) of the light guide 653 is preferably equal to or smaller than 10 mm. The light guide 653 is disposed in the housing 1. In the embodiment, the light guide 653 is disposed in the second case 12. More specifically, the light guide 653 is disposed in the through hole formed in the bottom wall 310 of the holder 31. A front surface of the light guide 653 is exposed to the recessed space S1. The front surface of the light guide 653 constitutes a part of the bottom surface 190 of the recess 19. According to the hair control device 10 of the present embodiment, the light guide 653 is disposed in a part of the bottom surface 190 of the recess 19 of the housing 1.

The imaging device 651 includes a camera including an image sensor, for example. The image sensor includes a two-dimensional image sensor such as a Charge Coupled Devices (CCD) image sensor, a Complementary Metal-Oxide Semiconductor (CMOS) image sensor, or the like, for example. The imaging device 651 includes an optical system including a lens or the like. The optical system is configured to form an image of the incident light on an imaging surface of the image sensor. The image sensor is configured to covert the intensity of the incident light into electric signals. Accordingly, the imaging device 651 generates (takes) an image. The imaging device 651 may be configured to generate images sequentially (e.g., about 30 fps). Alternatively, the imaging device 651 may be configured to generate images intermittently (e.g., every second or every few seconds). Alternatively, the imaging device 651 may be configured to generate an image at a certain timing (e.g., when it is instructed by the controller 71). The imaging device 651 further includes a casing in which the image sensor and the optical system are housed.

The imaging device 651 is disposed in the housing 1. The imaging device 651 is disposed in the second case 12. The imaging device 651 is disposed in the housing recess 591 of the coupling member 59. The imaging device 651 is disposed in the housing 1 such that a shooting direction (an optical axis thereof) is directed forward (i.e., it takes an image of a front side of the housing 1).

The imaging device 651 is disposed in the housing 1 and located behind the light guide 653. The imaging device 651 faces a rear surface of the light guide 653. The light in the recessed space S1 will enter the light guide 653 through a front surface, and travels in the light guide 653 to be guided toward the imaging device 651. Accordingly, the imaging device 651 can take an image of an object (skin 92) present in a space in the recess 19 (i.e., the recessed space S1) or present in front of the recess 19. The imaging device 651 is disposed such that the recesses space S1 is contained in a viewing angle of the imaging device 651. In the embodiment, the imaging unit 65 is housed in the housing 1 so as to take an image of a front side of the bottom surface 190 of the recess 19. In short, a front surface of the light guide 653 serves as the light incident surface 650 of the imaging unit 65 that includes the imaging device 651 and the light guide 653. The light incident surface 650 of the imaging unit 65 faces substantially in parallel to the surface of the skin 92 introduced into the recessed space S1. The optical axis of the imaging unit 65 is substantially perpendicular to the surface of the skin 92 to be taken the image.

According to the hair control device 10 of the present embodiment, the light guide 653 includes the light incident surface 650. The light guide 653 and the imaging device 651 are arranged in an order of: the bottom surface 190 of the recess 19 of the housing 1, the light guide 653, and the imaging device 651. The imaging device 651 is disposed apart from the recessed space S1, which can prevent the dew condensation on the surface of the imaging device 651.

As described above, the through hole is formed in the center in the Y-axis of the bottom wall 310 of the holder 31. Moreover, the imaging unit 65 (light guide 653) is disposed in the through hole. Therefore, the light incident surface 650 of the imaging unit 65 is located in the part of the housing 1 corresponding to the bottom surface 190 of the recess 19 to be positioned around the center in the longitudinal axis (Y-axis) of the recess 19. According to this location of the imaging unit 65, the recessed space S1 is easily contained in the viewing angle of the imaging unit 65. That is, the light incident surface 650 of the imaging unit 65 is positioned around the center in the longitudinal axis of the recess 19 having the recessed groove shape, and thus an image of a large (in the present embodiment, substantially a whole) area of the recessed space S1 can be taken by a single imaging unit 65.

The imaging light source 652 is disposed to illuminate an imaging area of the imaging device 651. The position of the imaging light source 652 is not particularly limited. The imaging light source 652 may be disposed in the bottom wall 310 of the holder 31 (i.e., in the part of the bottom surface 190 of the recess 19 of the housing 1), or may be disposed in the substrate 21 of the light source unit 20, for example.

The imaging unit 65 (imaging device 651) is configured to transmit the taken image to the controller 71. The imaging device 651 is connected to the controller 71 and the power source 72 with a wiring (power line and telecommunications line) passing through the coupling member 59 and transmits the image to the controller 71 via the wiring (telecommunications line).

The thermal insulation part 66 thermally insulates the imaging unit 65 from the outer environment. In the embodiment, the thermal insulation part 66 thermally insulates the light guide 653 from the outer environment. The thermal insulation part 66 has the thermal insulation property. The thermal insulation part 66 may has an air layer thereinside, for example. The thermal insulation part 66 may be made of the material having thermal insulation property, such as the foamed plastic. As shown in FIG. 28, the thermal insulation part 66 is disposed to surround a peripheral surface (surfaces other than a front surface and rear surface) of the light guide 653.

Specifically, the thermal insulation part 66 is disposed between the light guide 653 and the light transmission member 3. The thermal insulation part 66 thermally insulates the light guide 653 from the light transmission member 3. As described above, the light transmission member 3 is cooled by the cooler 5. If the light guide 653 is thermally connected to the light transmission member 3, then the light guide 653 is also cooled by the cooler 5 and the temperature of the light guide 653 is decreased. When the temperature of the light guide 653 decreases, water droplet may appear on a surface of the light guide 653 exposed outward (i.e., on the front surface; the light incident surface 650) by the dew condensation depending on the temperature and the humidity of the surrounding environments. When the water droplet appears on the front surface of the light guide 653, the light to be incident on the front surface of the light guide 653 may be refracted or scattered by the water droplet. In this case, a desired image of the recessed space S1 may not be able to be taken by the imaging device 651. In this regard, according to the hair control device 10 of the present embodiment including the thermal insulation part 66, the light guide 653 is less likely to be excessively cooled. This can prevent the dew condensation water from appearing on the surface of the light guide 653. That is, the hair control device 10 of the present embodiment includes the thermal insulation part 66 thermally insulating the imaging unit 65 (the light guide 653) from a cooling member (the light transmission member 3). This can prevent the dew condensation water from appearing on the light incident surface 650 of the imaging unit 65.

Moreover, the thermal insulation part 66 thermally insulates the light guide 653 from the coupling member 59. This can prevent the cooling of the light guide 653. This can prevent the dew condensation water from appearing on the surface of the light guide 653.

The controller 71 is configured to control the operation of the hair control device 10. The controller 71 is configured to control the operations of the light emitting unit 2, the cooler 5, the imaging unit 65 (the imaging device 651 and the imaging light source 652), and the communications unit 73.

The controller 71 controls the operations of the light emitting unit 2, the cooler 5, the imaging unit 65, and the communications unit 73 in accordance with the operation given to the operations unit 6, for example.

As shown in FIG. 30, the controller 71 includes an imaging controller 711, an analyzing unit 712, a lighting controller 713, a cooling controller 714, and a communications controller 715.

The imaging controller 711 controls the operation of the imaging unit 65. The imaging controller 711 starts the imaging unit 65 (imaging device 651) taking photos in accordance with the operation (start operation) given to the operations unit 6, for example. The imaging unit 65 takes photos to generate the images at appropriate timings. For example, the imaging unit 65 takes a photo to generate an image when it is detected that an object (skin 92) is contacted with the front surface of the housing 1 of the photocosmetic device 100 (e.g., a front surface of the holder 31). The contact of the object with the front surface of the housing 1 may be detected by a contact sensor provided to the front surface of the housing 1, for example. For example, the imaging unit 65 (imaging device 651) is configured to come into focus on an entrance opening of the recessed space S1, and has a comparatively large depth of field. The imaging unit 65 thus can take an image at a position of the entrance opening of the recessed space S1. The imaging unit 65 (imaging device 651) may have a function of autofocus. In this case, the imaging unit 65 (imaging device 651) may be configured to automatically focus on the subject (surface of the skin 92) with reference to the distance to the subject. The images are taken when the photocosmetic device 100 is brought into contact with the skin 92 as described. However, the imaging unit 65 may be configured to keep only one image which is taken lastly before the illumination of the light, and other images may be discarded, for example. The imaging unit 65 transmits the obtained image to the controller 71. Alternatively, the imaging unit 65 may be configured to take photos to generate a plurality of images and to transmit the generated plurality of images to the controller 71. The controller 71 may be configured to associate the image(s) supplied from the imaging unit 65 with the imaging time(s) (times when the image is obtained) and to store them on a storing unit.

The imaging unit 65 takes images of an inside of the recessed space S1, as described above. Therefore, when the skin 92 is introduced into the recessed space S1, the imaging unit 65 generates images of the surface of the skin 92.

The analyzing unit 712 is configured to analyze a condition of the skin 92 based on the image taken by the imaging unit 65.

The analyzing unit 712 is configured to analyze the image obtained by the imaging unit 65. The analyzing unit 712 analyzes the image based on the image processing technique. The image processing performed by the analyzing unit 712 may include the image correction processing (such as the contrast correction), the image conversion processing (such as the binarization and the affine transformation), the image recognition (such as the pattern recognition) or the like.

The analyzing unit 712 is configured to perform the image processing to determine whether a region representing the skin 92 is contained in the image. When determining that the region representing the skin 92 is contained in the image, the analyzing unit 712 further determine whether a region representing an object (e.g., clothing) other than the skin 92 is contained in the image.

Moreover, when determining that the region representing the skin 92 is contained in the image, the analyzing unit 712 determines the condition of the skin 92. The condition of the skin 92 to be determined by the analyzing unit 712 includes a condition whether there is a portion (hereinafter, referred to as a "first portion") having a predetermined feature in the surface of the skin 92, for example. Examples of the predetermined feature include a feature indicative of a mole and a feature indicative of acne vulgaris. The predetermined feature may include a feature indicative of a nail, a feature indicative of a scab, or the like. That is, the analyzing unit 712 is configured to determine, based on the image taken by the imaging unit 65, a presence or absence of the first portion having the predetermined feature (such as the mole and the acne vulgaris) in the surface of the skin 92.

The image to be analyzed by the analyzing unit 712 is an image which is taken before (just before) the skin 92 is introduced into the recessed space S1, out of a plurality of images taken by the imaging unit 65. The image to be analyzed by the analyzing unit 712 is an image which is taken in a state where the surface of the skin 92 is located in the entrance of the recessed space S1, for example. The state where the surface of the skin 92 is located in the entrance of the recessed space S1 indicates, for example, a state where the surface of the skin 92 and an imaginal plane containing the entrance opening of the recessed space S1 are spatially coincide with each other. Whether the surface of the skin 92 is located in the entrance opening of the recessed space S1 may be determined based on the detection result of the contact sensor described above. Whether the surface of the skin 92 is located in the entrance opening of the recessed space S1 may alternatively be determined based on the positional relationship between the skin 92 and the light transmission member 3 (or the holder 31) in the image taken by the imaging unit 65. Whether the surface of the skin 92 is located in the entrance of the recessed space S1 may specifically be determined based on whether a front end of the light transmission member 3 is concealed by the skin 92 or not, for example.

The lighting controller 713 controls the operation of the light emitting unit 2. The lighting controller 713 controls the operation of the light emitting unit 2 based on the analysis result made by the analyzing unit 712.

The lighting controller 713 of the present embodiment can control the lighting of the light emitting unit 2 by the LED 221 as one unit, not by the light emitting unit 2 nor by the light source unit 20. In other words, the lighting controller 713 of the present embodiment is configured to individually control the operations of a plurality of LEDs 221 of the light emitting unit 2.

Based on the analysis result by the analyzing unit 712, the lighting controller 713 causes one or more LEDs 221 that faces the skin 92 to emit light, among the plurality of LEDs 221. In other words, the lighting controller 713 causes a LED(s) 221 that faces the object (e.g., clothing) other than the skin 92 not to emit light, among the plurality of LEDs 221. This can prevent the emission of the unnecessary light that does not contribute to the treatment of the skin 92. Alternatively, the lighting controller 713 may cause a LED(s) 221 that faces the object other than the skin 92 to emit light with the intensity smaller than that of the light emitted from the one or more LEDs 221 facing the skin 92.

Based on the analysis result by the analyzing unit 712, the lighting controller 713 controls the operation of the light emitting unit 2 such that the illumination intensity of the light to the portion having the predetermined feature in the surface of the skin 92 (i.e., first portion) is smaller than the illumination intensity of the light to a portion other than the first portion in the surface of the skin 92 (hereinafter, referred to as a "second portion"). Specifically, the lighting controller 713 controls the operations of the plurality of LEDs 221 such that the intensity of the light of one or more LEDs 221 facing the first portion of the surface of the skin 92 is smaller than the intensity of the light of another one or more LEDs 221 facing the second portion of the surface of the skin 92. That is, the lighting controller 713 is configured to control the operation of the light emitting unit 2 such that when the first portion is present in the surface of the skin 92, an exposure amount of the light from the light emitting unit 2 to the first portion in the surface of the skin 92 is smaller than an exposure amount of the light from the light emitting unit 2 to the second portion in the surface of the skin 92. For example, the lighting controller 713 may control the operations of the plurality of LEDs 221 such that the intensity of the light of one LED 221 facing the first portion is smaller than the intensity of the light of another one LED 221 facing the second portion. When the predetermined feature indicates the mole, the acne vulgaris or the scab, the portion of the skin having the predetermined feature has the comparatively large absorbance. Therefore, the light is likely to be more absorbed in the first portion than in the second portion. Therefore, the temperature of the skin 92 is likely to increase around the first portion compared to the second portion, which may stimulate the nerves that detect the pain in the skin 92 to cause the uncomfortable feeling in a user. In this regard, the hair control device 10 of the present embodiment controls the operation of the light emitting unit 2 such that the exposure amount of the light to the first portion is smaller than the exposure amount of the light to the second portion in the surface of the skin 92 as described above. As a result, the uncomfortable feeling to be caused in a user can be reduced. When the predetermined feature indicates the nail or the scab, no hair 91 will grow from the portion having the predetermined feature. No light illumination is therefore necessary by the hair control device 10. Moreover, the hair control device 10 of the present embodiment can reduce the power consumption, since the operation of the light emitting unit 2 is controlled such that the exposure amount of the light to the first portion is smaller than the exposure amount of the light to the second portion in the surface of the skin 92.

As described above, according to the hair control device 10 of the present embodiment, the lighting controller 713 controls the operation of the light emitting unit 2 based on the analysis result by the analyzing unit 712. It is therefore possible to illuminate the skin 92 with light suitable for the condition of the skin 92.

The lighting controller 713 may controls, based on the analysis result by the analyzing unit 712, the operation of the light emitting unit 2 such that the intensity of the light to a region including the hair 91 in the surface of the skin 92 is greater than the intensity of the light to a region of no hair 91 in the surface of the skin 92. Treatment efficiency can be improved.

The lighting controller 713 may be configured to, based on the analysis result by the analyzing unit 712, adjust the intensity of the light emitted from the light emitting unit 2 to the skin 92 depending on the stage (whether it is in the early stage of the anagen or the later stage of the anagen or the catagen or the telogen) of the hair cycle of the hair 910 as the illumination target.

The hair control device 10 may provide an alert to the user when it is determined that the first portion is present in the surface of the skin 92 based on the analysis result by the analyzing unit 712.

The lighting controller 713 stops the light emitting unit 2 emitting the light in accordance with the operation (stop operation) given to the operations unit 6, for example.

The cooling controller 714 controls the operations of the cooler 5 (the cooling fan 51 and the Peltier device 52). As described above, the cooling controller 714 controls, based on the measurement result of the temperature sensor, the operation of the cooler 5 such that the temperature of the surface of the skin 92 falls within the range of -5°C to 35°C, for example. However, the cooling controller 714 is not limited to this, but may control the operation of the cooler 5 in accordance with the operation (such as the operation to turn on or off the cooler 5) given to the operations unit 6.

The communications controller 715 controls the operation of the communications unit 73.

The communications unit 73 includes a communications module to communicate with an external device. The communications unit 73 is configured to communicate with the terminal device 99 provided externally.

As shown in FIG. 30, the communications unit 73 is configured to be connected to a communications network N1 and has a function to perform communications through the communications network N1. The communications network N1 may include the Internet, a telephone network, and the like. The communications network N1 may include a network compliant with a single communication protocol, or may include multiple networks compliant with mutually different communications protocols. The communication protocol may be selected from various known wired or wireless communication standards. Although not explicitly shown in FIG. 30, the communications network N1 may include data communication equipment such as repeater hubs, switching hubs, bridges, gateways, routers, and the like.

The communications unit 73 is in compatible with a predetermined communication protocol. The predetermined communication protocol for the communications unit 73 may be selected from various known wired or wireless communication standards. The predetermined communication protocol for the communications unit 73 may be a protocol suitable for short range wireless communication (such as a protocol for Wi-fi (trademark) or Bluetooth (trademark)), for example.

The communications controller 715 controls the communications unit 73 to make the communications unit 73 output, to the terminal device 99, the image taken by the imaging unit 65. The image output through the communications unit 73 may include an image which is taken before (just before) the skin 92 is introduced into the recessed space S1, in other words, an image which is obtained before (just before) the light of the light emitting unit 2 is illuminated to the skin 92. However, the image output through the communications unit 73 is not limited to this, but may include an image which is taken after the light of the light emitting unit 2 is illuminated to the skin 92, or may include all of a plurality of images taken by the imaging unit 65.

If the hair control device 10 is used one time everyday by a user, the communications controller 715 outputs the image taken by the imaging unit 65 before or after the use of the hair control device 10 in each day, for example.

In short, the communications unit 73 has a function to output, to the terminal device 99, image information relating to the image taken by the imaging unit 65. The image information may include the image taken by the imaging unit 65.

### (2.2.2) Photocosmetic System

Next, the photocosmetic system 1000 of the present embodiment is described. As described above, the photocosmetic system 1000 includes the photocosmetic device 100 (hair control device 10) and the terminal device 99.

The terminal device 99 is an information terminal. The terminal device 99 is a mobile device to be carried by the user, for example. The terminal device 99 is a smartphone, for example. However, the terminal device 99 is not limited to the smartphone, but may be a mobile information terminal such as a tablet computer or a wearable computer, or a personal computer (such as a desktop computer or a laptop computer). The terminal device 99 is not limited to a general purpose device but may be a dedicated device.

As shown in FIG. 30, the terminal device 99 includes a controller 991, a display unit 992, and a communications unit 993.

The controller 991 is implemented as a microcontroller including one or more processors and one or more memories. The microcontroller performs the function of the controller 991 by making the one or more processors execute a program stored in the one or more memories. The program may be stored in advance in the memories, or may be downloaded via a telecommunications line or distributed after having been stored in a non-transitory storage medium such as a memory card. In other words, the program is a program for making the one or more processors function as the controller 991.

The controller 991 performs the control on a whole functions of the terminal device 99, that is, controls the display unit 992 and the communications unit 993. The function of the controller 991 is realized by the one or more processors of the controller 991 executing the program.

The communications unit 993 includes a communications module to communicate with the hair control device 10 (photocosmetic device 100). The communications unit 993 is in compatible with the predetermined communication protocol described above.

The communications unit 993 receives the image output from the hair control device 10. The image received by the communications unit 993 is the image taken by the imaging unit 65 of the hair control device 10.

If the hair control device 10 is used one time everyday by a user, the communications unit 993 receives the image from hair control device 10 before or after the use by the user of the hair control device 10 in each day, for example. That is, the terminal device 99 has a function to receive, from the communications unit 73 of the photocosmetic device 100, the image information relating to a plurality of images taken by the imaging unit 65 at various timings (e.g., taken everyday).

The terminal device 99 is configured to associate the plurality of images supplied from the hair control device 10 with received times (or times when the images are generated by the captions by the imaging unit 65) and store them in the storage unit.

The display unit 992 is used for displaying various information. The display unit 992 includes a flat-panel display such as a liquid crystal display and an organic EL display, for example. The terminal device 99 may include an input unit (such as a touchpad) configured to receive an operation input from a user. The touchpad of the input unit and the display of the display unit 992 may constitute a touchscreen panel.

The display unit 992 displays image relating information relating to the image supplied from the hair control device 10, for example.

For example, the display unit 992 displays the image supplied from the hair control device 10. That is, the terminal device 99 has a function to display, on the display unit 992, the image supplied from the hair control device 10. For example, the terminal device 99 displays two or more images on the display unit 992 in time series based on the information of times respectively associated with the two or more images. The terminal device 99 may display all the received images on the display unit 992 in one time, or may display one or more images selected from the received images. For example, the terminal device 99 may display in time series two or more images that have been taken with predetermined intervals (e.g., every week or every half month or every month or every three months). In short, the terminal device 99 has a function to display, in time series on the display unit 992, two or more images selected from the plurality of images represented by the received image information.

The terminal device 99 further has a function to compute, based on the image supplied from the hair control device 10, skin condition information representing the condition of the skin 92 by numerical values. The skin condition information used herein may indicate information that can be computed based on a plurality of images taken by the imaging unit 65 at different timings. Examples of the skin condition information include a remaining hair ratio and a hair removal ratio. The remaining hair ratio indicates a ratio of: the number of remaining hairs per a unit area when the image is taken; to the number of hairs per a unit area in the initial state. The hair removal ratio indicates the ratio of: the removed number of hairs per a unit area when the image is taken; to the number of hairs per a unit area in the initial state. For example, the hair removal ratio (%) = 100 - the remaining hair ratio (%). The skin condition information is not limited to the remaining hair ratio or the hair removal ratio, but may be a numerical value representing the condition of the skin 92 not related to the hair 91 (such as the degree of the skin texture), for example.

The terminal device 99 may display the images supplied from the hair control device 10 together with the skin condition information in time series. When the user see the display, the user can easily understand the usefulness of the device.

### (2.3) Variations of Embodiment 2

Variations of the embodiment 2 will be described. In the following description, the embodiment 2 described above may be referred to as a "basic example (of the embodiment 2)". In the description of the variations, components that are substantially the same as those of a basic aspect may be appropriately omitted. The basic example and the variations may be readily modified depending on a design choice or any other factor, without departing from a true spirit and scope of the present disclosure.

### (2.3.1) First Variation

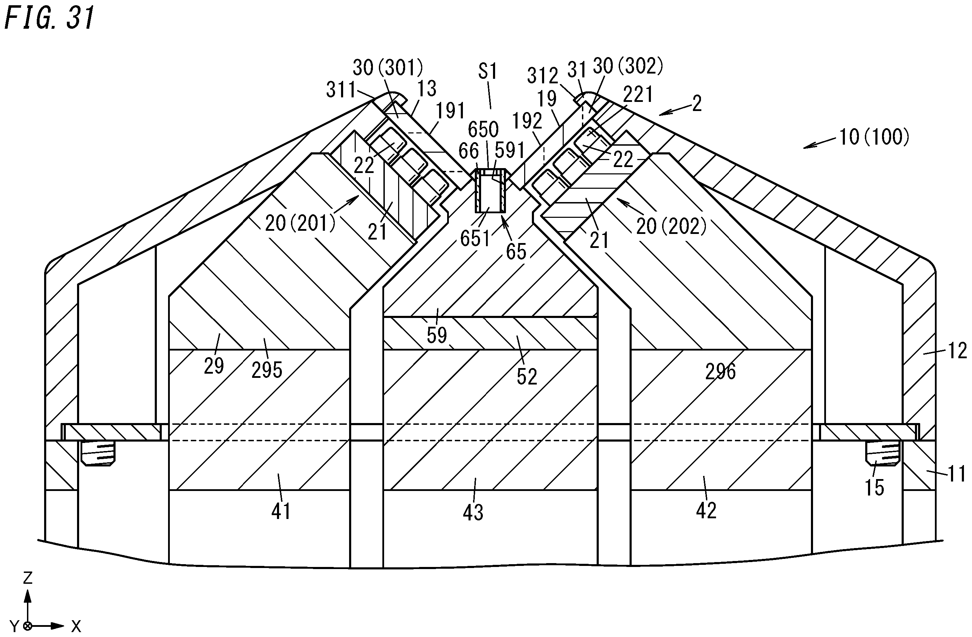

A hair control device 10 (photocosmetic device 100) of the present variation will be described with reference to FIG. 31. The hair control device 10 of the present variation differs from the hair control device 10 of the basic example in a structure of an imaging unit 65.

As shown in FIG. 31, according to the hair control device 10 of the present variation, the imaging unit 65 does not include a light guide 653. An imaging device 651 is disposed in a housing recess 591 of a coupling member 59. The imaging device 651 has a front surface (specifically, a lens of an optical system) that is exposed to the recessed space S1. According to the hair control device 10 of the present variation, therefore, the front surface of the imaging device 651 serves as a light incident surface 650 of the imaging unit 65. In other words, the imaging device 651 including an image sensor has the light incident surface 650. The imaging unit 65 is configured to take an image of a space (recessed space S1) inside a recess 19 based on light entering through the light incident surface 650 (the front surface of the imaging device 651).

Moreover, a thermal insulation part 66 is disposed to surround a peripheral surface (surfaces other than a front surface and a rear surface) of the imaging device 651.

According to the hair control device 10 of the present variation, an image taken region of the imaging unit 65 and a region to which the light of the light emitting unit 2 is illuminated surely coincide with each other with high reliability. This allows the light of the light emitting unit 2 to be illuminated on a desired region of the surface of the skin 92 with a high reliability, as a result of which the decline in the treatment efficiency to treat the skin 92 can be reduced.

The hair control device 10 of the present variation can reduce the volume of the housing recess 591 by the size of the light guide 653, compared to the hair control device 10 of the basic example. This can increase the volume of the coupling member 59 and thus increase the heat capacity of the coupling member 59, which can facilitate the cooling of the coupling member 59 by the cooler 5. The power consumption of the cooler 5 thus can be reduced.

### (2.3.2) Second Variation

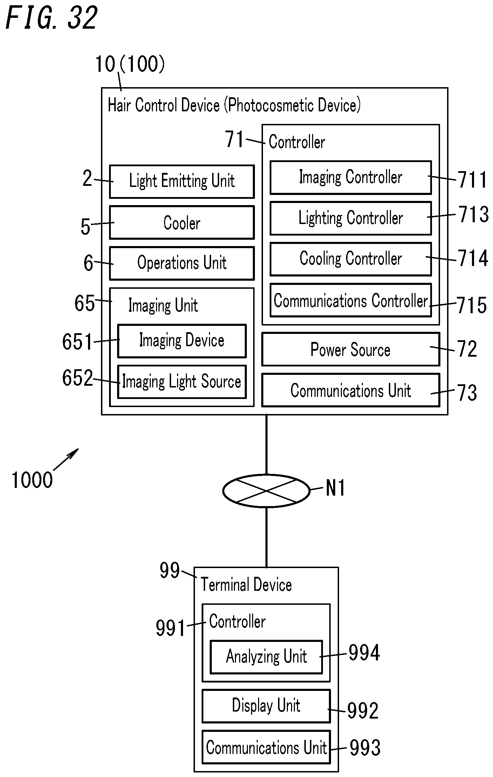

A photocosmetic system 1000 including a hair control device 10 (photocosmetic device 100) of the present variation will be described with reference to FIG. 32. The photocosmetic system 1000 of the present variation differs from the photocosmetic system 1000 of the basic example mainly in a configuration of a controller 991 of a terminal device 99.

According to the photocosmetic system 1000 of the present variation, a controller 71 of the hair control device 10 (photocosmetic device 100) does not include an analyzing unit 712 but a controller 991 of the terminal device 99 includes an analyzing unit 994.

According to the photocosmetic system 1000 of the present variation, when the imaging unit 65 starts taking an image in accordance with the operation (start operation) given to the operations unit 6, the communications unit 73 of the hair control device 10 transmits, to the terminal device 99 via a communications network N1, information relating to the image taken by the imaging unit 65 (hereinafter, referred to as "first information").

When the terminal device 99 receives the first information from the hair control device 10 via the communications unit 993, the analyzing unit 994 analyzes the condition of the skin 92 based on the image included in the received first information. The terminal device 99 transmits, from the communications unit 993 via the communications network N1 to the hair control device 10, information about the analysis result made by the analyzing unit 994 (hereinafter, referred to as "second information").

The lighting controller 713 of the hair control device 10 controls the operation of the light emitting unit 2 based on the second information supplied from the terminal device 99.

In short, according to the hair control device 10 of the photocosmetic system 1000 of the present variation, the communications unit 73 has: a function to output, to the terminal device 99, the first information relating to the image taken by the imaging unit 65; and a function to receive, from the terminal device 99, the second information relating to the condition of the skin 92 analyzed based on the image of the first information. The lighting controller 713 is configured to control the operation of the light emitting unit 2 based on the second information supplied from the terminal device 99.

According to the photocosmetic system 1000 of the present variation, it is possible to illuminate the skin 92 with light suitable for the condition of the skin 92.

In an alternative example, the controller 71 of the hair control device 10 may also include the analyzing unit 712.

Moreover, the functions of an imaging controller 711, a lighting controller 713, a cooling controller 714 and an operations unit 6 may be provided to the terminal device 99.

### (2.3.3) Third Variation

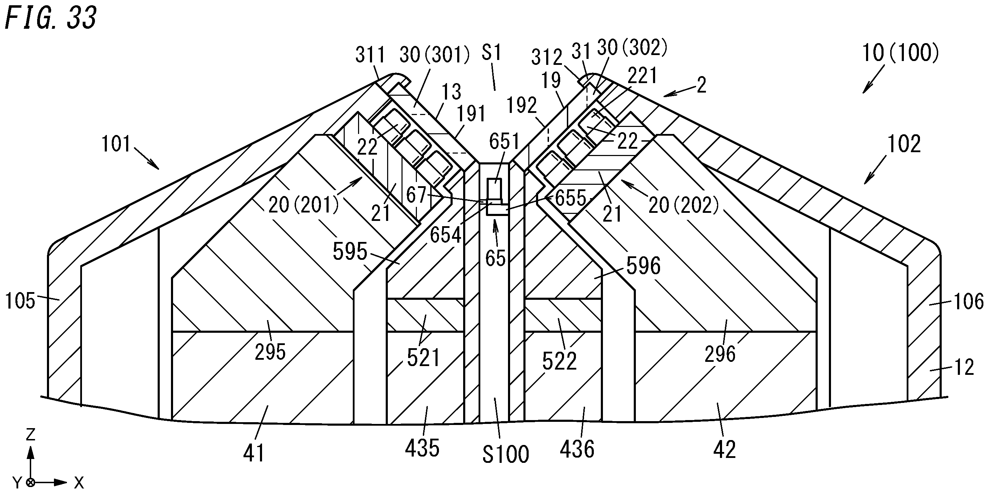

A hair control device 10 (photocosmetic device 100) of the present variation will be described with reference to FIG. 33. The hair control device 10 of the present variation differs from the hair control device 10 of the basic example in a configuration of a housing 1, a heat dissipation part 4, an imaging unit 65 etc.

As shown in FIG. 33, according to the hair control device 10 of the present variation, the housing 1 includes a first housing part 105 in which a first light source unit 201 is housed and a second housing part 106 in which a second light source unit 202 is housed. The first housing part 105 and the second housing part 106 are disposed separately (separated) from each other in a direction in which the first light source unit 201 and the second light source unit 202 are arranged. The hair control device 10 of the present variation is not provided with a Peltier device 52, a third heat dissipation part 43 and a coupling member 59.

Specifically, the hair control device 10 of the present variation includes a first light source block 101 and a second light source block 102.

The first light source block 101 includes the first light source unit 201, a first light transmission piece 301, a first holding member 295, a first heat dissipation part 41, and the first housing part 105. The first housing part 105 has a box shape and houses therein the first light source unit 201, the first holding member 295, and the heat dissipation part 41. The first housing part 105 holds the first light transmission piece 301 such that the first light transmission piece 301 is positioned on an optical axis of the first light source unit 201.

The second light source block 102 includes the second light source unit 202, a second light transmission piece 302, a second holding member 296, a second heat dissipation part 42, and the second housing part 106. The second housing part 106 has a box shape and houses therein the second light source unit 202, the second holding member 296, and the heat dissipation part 42. The second housing part 106 holds the second light transmission piece 302 such that the second light transmission piece 302 is positioned on an optical axis of the second light source unit 202.

According to the hair control device 10 (photocosmetic device 100) of the present variation, at least one (in the variation, both) of the first light source block 101 or the second light source block 102 is movable in a direction (direction along the X-axis) in which the first light source unit 201 and the second light source unit 202 are arranged.

An imaging device 651 of the imaging unit 65 is disposed in a gap (space S100 communicated with to a recessed space S1) between the first housing part 105 and the second housing part 106.

The imaging device 651 is placed on a supporting board 654. The supporting board 654 is fixed to a stationary part 655 which is fixed to either of the first housing part 105 or the second housing part 106 (in the variation, to the second housing part 106). Accordingly, the imaging device 651 is positionally fixed to the housing 1 (to the second housing part 106).

According to the hair control device 10 of the present variation, the imaging device 651 is disposed in the space S100 communicated with the recessed space S1. It is accordingly possible to confirm that the skin 92 is correctly introduced into the recessed space S1 by checking the image taken by the imaging unit 65.

As shown in FIG. 33, the hair control device 10 of the present variation further includes a sensing unit 67.

The sensing unit 67 is configured to detect that the first housing part 105 and the second housing part 106 are in a close state. The sensing unit 67 is provided to the supporting board 654 or a housing part (the first housing part 105 or the second housing part 106). In the variation, the sensing unit 67 is provided to a side wall of the first housing part 105.

The sensing unit 67 includes a switch or a sensor. The switch of the sensing unit 67 includes a mechanical switch, for example. The sensor of the sensing unit 67 includes a photosensor (photo diode) configured to detect whether the sensor is in contact with the supporting board 654 based on the sensed amount of light, or a pressure sensor configured to detect a pressure applied when the sensor is pressed by the supporting board 654, or a capacitive sensor configured to sense the capacitance between the sensor and the supporting board 654, for example.

With the sensing unit 67, it is possible to detect whether the first housing part 105 and the second housing part 106 are in the close state (whether the space S100 is closed most).

In an alternative example of the hair control device of the present variation, a Peltier device may be provided to the first light source unit 201 and/or the second light source unit 202.

### (2.3.4) Fourth Variation

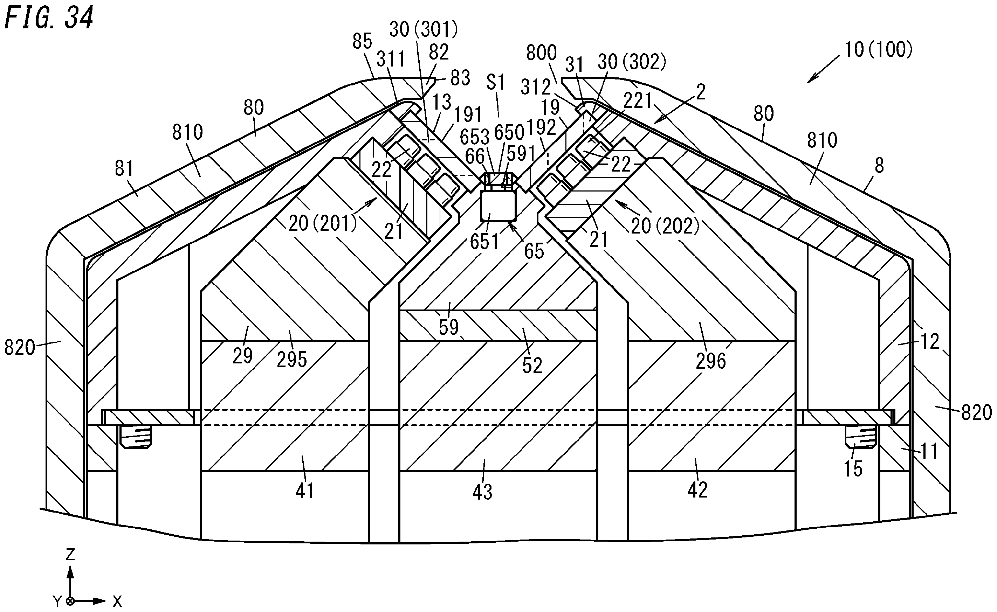

A hair control device 10 (photocosmetic device 100) of the present variation will be described with reference to FIG. 34. The hair control device 10 of the present variation differs from the hair control device 10 of the third variation in further including a skin introducing unit 8.

As shown in FIG. 34, the skin introducing unit 8 has an opening 800 and is configured to introduce the skin 92 through the opening 800 into a recessed space S1 surrounded by a light exit surface 13. The skin introducing unit 8 includes a pinching unit 80. The configuration of the skin introducing unit 8 is substantially the same as that in the hair control device 10 of the embodiment 1 and thus the explanations thereof will be omitted.

The hair control device 10 of the present variation has the advantage of improving the treatment efficiency for treating the skin 92 as with the hair control device 10 of the embodiment 1, in addition to the advantage of reducing the decline in the treatment efficiency to treat the skin 92 as with the hair control device 10 of the embodiment 2.

### (2.3.5) Other Variations

In one variation, a housing 1 does not have to house therein an entire of a light emitting unit 2. A housing 1 may house a light emitting unit 2 with a part of a light emitting unit 2 (e.g., a part of a substrate 21) protruding from the housing 1, for example.

In one variation, a housing 1 does not have to house therein an entire of an imaging unit 65. A housing 1 may house an imaging unit 65 with a part of an imaging unit 65 (e.g., a part of a light guide 653) protruding from the housing 1, for example.

In one variation, a substrate 21 and a light transmission member 3 (a first light transmission piece 301 or a second light transmission piece 302) may not have to be in parallel to each other, but may be inclined. In this case, an inclined angle between a light transmission member 3 (light transmission piece 30) and a substrate 21 may be set in consideration with a loss of light caused by the total refection. The inclined angle between the light transmission piece 30 and the substrate 21 may be set such that (majority) of the light emitted from the light source 22 and traveling in the light transmission piece 30 enters a light exit surface 13 (front surface of the light transmission piece 30) by an incident angle equal to or smaller than the critical angle. The critical angle means the smallest angle of incidence that yields total reflection. The critical angle depends on the refractive index of the material of the light transmission piece 30 and the refractive index of the air. Disposing the substrate 21 and the light transmission member 3 in this manner can reduce the loss of light caused by the total reflection, and makes it easy to illuminate the desired amount of the light on the skin 92.

In one variation, a light source unit 20 may include an optical member configured to change a direction of an optical path of light of a light source 22 by the reflection or the refraction, such as a mirror, a half mirror, or a prism. In this case, an optical axis of the light source unit 20 may not coincide with the optical axis of the light source 22.

In one variation, a controller 71 may control lighting of a light emitting unit 2 by a light source unit 20 as one unit or the light emitting unit 2 as one unit. In this case, the lighting controller 713 may be configured to, when determining that there is a first position in a surface of a skin 92 based on an image taken by an imaging unit 65, control the light emitting unit 2 to decrease the intensity of light of the light emitting unit 2 compared to a case where there is no first portion.

In one variation, a light guide 653 is not limited to a glass plate, but may be a lens, for example. When the light guide 653 is a lens, the light guide 653 may have a height greater than 10 mm.

In one variation, a light guide 653 and an imaging device 651 may be apart from each other in the Z-axis. Nevertheless the imaging device 651 and the light guide 653 are apart from each other in the Z-axis, the dew condensation hardly occurs on a surface of the imaging device 651 when the space in which the imaging device 651 is disposed is hermetically sealed.

In one variation, a portion (first portion) having a predetermined feature may include a portion where the hair 91 is not completely removed from the skin 92. If light is illuminated to a region of the skin 92 where the hair 91 is not completely removed, sufficient amount of light may not reach the deep part of the skin 92. When there is a portion where the hair 91 is not completely removed from the skin 92, a notification may be provided to a user. This can reduce the decline in the treatment efficiency to treat the skin 92.

In one variation, a condition of the skin 92 to be determined by an analyzing unit 712 is not limited to a condition whether a first portion is present in a surface of the skin 92, but may include, for example, the number of hairs 91 (e.g. the number of hairs 91 per unit area) and the like.

In one variation, a light transmission member 3 (light transmission piece 30) may have a shape serving as a lens.

In one variation, a photocosmetic device 100 may not include a light transmission member 3.

In one variation, the number of light source units 20 included in a light emitting unit 2 is not limited to two, but may be three or more.

### (3) Other Configurations

The photocosmetic device 100 and the photocosmetic system 1000 according to the present disclosure includes, in its controller 71 and its controller 991 for example, a computer system. In that case, the computer system may include, as principal hardware components, a processor and a memory. The functions of the controller 71 or the controller 991 may be performed by making the processor execute a program stored in the memory of the computer system. The program may be stored in advance in the memory of the computer system. Alternatively, the program may also be downloaded through a telecommunications line or be distributed after having been recorded in some non-transitory storage medium such as a memory card, an optical disc, or a hard disk drive, any of which is readable for the computer system. The processor of the computer system may be made up of a single or a plurality of electronic circuits including a semiconductor integrated circuit (IC) or a large-scale integrated circuit (LSI). As used herein, the "integrated circuit" such as an IC or an LSI is called by a different name depending on the degree of integration thereof. Examples of the integrated circuits include a system LSI, a very large-scale integrated circuit (VLSI), and an ultra large-scale integrated circuit (ULSI). Optionally, a field-programmable gate array (FPGA) to be programmed after an LSI has been fabricated or a reconfigurable logic device allowing the connections or circuit sections inside of an LSI to be reconfigured may also be adopted as the processor. Those electronic circuits may be either integrated together on a single chip or distributed on multiple chips, whichever is appropriate. Those multiple chips may be integrated together in a single device or distributed in multiple devices without limitation. As used herein, the "computer system" includes a microcontroller including one or more processors and one or more memories. Thus, the microcontroller may also be implemented as a single or a plurality of electronic circuits including a semiconductor integrated circuit or a largescale integrated circuit.

Also, the photocosmetic device 100 and the photocosmetic system 1000 are not limited to the configuration where the plurality of constituent elements (or the functions) of the controller 71 or the controller 991 are integrated together in a single housing. Alternatively, those constituent elements (or functions) of the controller 71 or the controller 991 may be distributed in multiple different housings. Still alternatively, at least some functions of the controller 71 or the controller 991 may be implemented as a cloud computing system as well.

The photocosmetic device 100 (body hair treatment device) may be a hair growth promoting device suitable for the hair growth promotion.

The photocosmetic device 100 may be a device suitable for improving the firmness or the wrinkles of the skin 92. For example, the effect for improving the firmness or the wrinkles can be achieved by irradiating the skin 92 with the light having the wavelength falling within the range of 600 nm to 800 nm to focus the light on the fibroblast present in the dermic layer (0.1 mm to 5 mm depth from the surface) of the skin 92 to promote the collagen production. A light source 22 of a light emitting unit 2 may be used as a light source for the improvement of the firmness or the wrinkles of the skin. Alternatively, the photocosmetic device 100 may include another light source for the improvement of the firmness or the wrinkles of the skin.

The photocosmetic device 100 may be a device suitable for improving (reducing) the blemishes/spots on the skin 92. For example, the effect for improving the blemishes/spots can be achieved by irradiating the skin 92 with the light having the wavelength falling within the range of 600 nm to 1000 nm to focus the light on the melanocytes present in the lower part of the epidermal layer (about 0.1 mm depth from the surface) of the skin 92 to prevent the melanin-production by the heat load on the melanocytes. A light source 22 of a light emitting unit 2 may be used as a light source for the improvement of the blemishes/spots. Alternatively, the photocosmetic device 100 may include another light source for the improvement of the blemishes/spots.

The photocosmetic device 100 may be a device suitable for slimming body. For example, the effect for slimming the body can be achieved by irradiating the skin 92 with the light having the wavelength falling within the range of 800 nm to 1200 nm to focus the light on the body fat in fatty layer (about 5 mm or more depth from the surface) of the skin 92 to heat the body fat to promote the absorption. A light source 22 of a light emitting unit 2 may be used as a light source for slimming the body. Alternatively, the photocosmetic device 100 may include another light source for the slimming the body.

The photocosmetic device 100 may be used only for the cosmetic purpose other than the body hair treatment (e.g., the improvement of the firmness or the wrinkles of the skin, the improvement of the blemishes/spots, or the slimming body). That is, the photocosmetic device 100 of the present disclosure is not limited to the body hair treatment device.

### (4) Aspect

As apparent from the embodiments and the variations described above, the present disclosure discloses the aspect below.

A photocosmetic device (100) of a first aspect includes a light emitting unit (2), a housing (1), and a light exit surface (13). The light emitting unit (2) is configured to generate light to be illuminated on a skin (92). The light emitting unit (2) is housed in the housing (1). The light of the light emitting unit (2) is emitted from the light exit surface (13) to an outside of the housing (1). The light exit surface (13) has a recessed shape recessed inward the housing (1). The photocosmetic device (100) further includes a skin introducing unit (8). The skin introducing unit (8) has an opening (800). The skin introducing unit (8) is configured to introduce the skin (92) through the opening (800) into a recessed space (S 1) surrounded by the light exit surface (13).

This aspect can facilitate to illuminate the light on the skin (92) in a state where the skin (92) is introduced into the recessed space (S1). The treatment efficiency for treating the skin (92) can be improved accordingly.

In a photocosmetic device (100) of a second aspect realized in combination with the first aspect, the skin introducing unit (8) includes a pinching unit (80) configured to pinch the skin (92) to introduce the skin (92) into the recessed space (S 1).

This aspect can facilitate to illuminate the light on the skin (92) in a state where the skin (92) is introduced into the recessed space (S1). The treatment efficiency for treating the skin (92) can be improved accordingly.

In a photocosmetic device (100) of a third aspect realized in combination with the second aspect, the pinching unit (80) is movable relative to the housing (1) along a moving axis (A1) so as to change an opening size of the opening (800).

This aspect can facilitate to illuminate the light on the skin (92) in a state where the skin (92) is introduced into the recessed space (S1). The treatment efficiency for treating the skin (92) can be improved accordingly.

In a photocosmetic device (100) of a fourth aspect realized in combination with the third aspect, the pinching unit (80) includes a base part (81) supported by the housing (1) to be movable relative to the housing (1) and a protruding part (82) protruding from the base part (81) along the moving axis (A1). The protruding part (82) has a tip end (83) constituting one edge of the opening (800). In an imaginal plane containing the moving axis (A1) and an opening direction of the opening (800), the protruding part (82) has at least one shape selected from a group consisting of a square bracket shape, an inclined surface shape, a semicircular shape, a quarter of a circle shape, an arc shape, and a curved surface shape including two or more curves having different curvatures.

This aspect can facilitate to illuminate the light on the skin (92) in a state where the skin (92) is introduced into the recessed space (S1). The treatment efficiency for treating the skin (92) can be improved accordingly.

In a photocosmetic device (100) of a fifth aspect realized in combination with the third aspect, the pinching unit (80) includes a protruding part (82) that faces the recessed space (S1). The protruding part (82) has a recessed surface (86) recessed away from the recessed space (S1).

This aspect can prevent the movement (slip) of the skin (92) inside the recessed space (S1) and thus can prevent the skin (92) from escaping (slipping out) from the recessed space (S1).

In a photocosmetic device (100) of a sixth aspect realized in combination with any one of the third to fifth aspects, the pinching unit (80) includes a base part (81) supported by the housing (1) to be movable relative to the housing (1) and a protruding part (82) protruding from the base part (81) along the moving axis (A1). The protruding part (82) has a tip end (83) constituting one edge of the opening (800). When viewed in an opening direction of the opening (800), the tip end (83) of the protruding part (82) has at least one shape selected from a group consisting of a straight line shape, a comb shape, a triangle wave shape, a reverse semicircular wave shape, and a circular wave shape.

This aspect can facilitate to illuminate the light on the skin (92) in a state where the skin (92) is introduced into the recessed space (S1). The treatment efficiency for treating the skin (92) can be improved accordingly.

In a photocosmetic device (100) of a seventh aspect realized in combination with any one of the third to sixth aspects, the pinching unit (80) includes a base part (81) supported by the housing (1) to be movable relative to the housing (1) and a protruding part (82) protruding from the base part (81) along the moving axis (A1). The protruding part (82) has a tip end (83) constituting one edge of the opening (800). The protruding part (82) has an inclined surface (84) that crosses the moving axis (A1) by an angle falling within the range of equal to or greater than 15° and equal to or smaller than 165°.

This aspect can facilitate to illuminate the light on the skin (92) in a state where the skin (92) is introduced into the recessed space (S1). The treatment efficiency for treating the skin (92) can be improved accordingly.

In a photocosmetic device (100) of an eighth aspect realized in combination with any one of the third to seventh aspects, a dimension (Y1) of the opening (800) measured along the moving axis (A1) in a state where the pinching unit (80) is to be moved to a position where the size of the opening (800) is the smallest falls within the range equal to or greater than 0 mm and equal to or smaller than 30 mm.

This aspect can facilitate to illuminate the light on the skin (92) in a state where the skin (92) is introduced into the recessed space (S1). The treatment efficiency for treating the skin (92) can be improved accordingly.

In a photocosmetic device (100) of a ninth aspect realized in combination with any one of the third to eighth aspects, the light exit surface (13) includes a first inclined surface (131) and a second inclined surface (132) facing each other in the moving axis (A1). The first inclined surface (131) and the second inclined surface (132) cross each other at a crossing angle (θ1) falling within the range greater than 0° and smaller than 180°.

This aspect can facilitate to illuminate the light on the skin (92) in a state where the skin (92) is introduced into the recessed space (S1). The treatment efficiency for treating the skin (92) can be improved accordingly.

A photocosmetic device (100) of a tenth aspect realized in combination with any one of the first to ninth aspects further includes a light transmission member (3) allowing the light of the light emitting unit (2) to pass through. The light exit surface (13) is at least partially constituted by a surface of the light transmission member (3).

This aspect can facilitate to illuminate the light on the skin (92) in a state where the skin (92) is introduced into the recessed space (S1). The treatment efficiency for treating the skin (92) can be improved accordingly.

In a photocosmetic device (100) of an eleventh aspect realized in combination with the tenth aspect, the light emitting unit (2) includes a light source (22) disposed to face the light transmission member (3).

This aspect can reduce the distance from the light source (22) to the skin (92).

In a photocosmetic device (100) of a twelfth aspect realized in combination with any one of the first to eleventh aspects, a depth (X1) falls within the range greater than 0 mm and equal to or smaller than 30 mm. The depth (X1) is measured as a distance from the opening

(800) to a far end part, most distant from the opening (800), of the light exit surface (13).

This aspect can facilitate to illuminate the light on the skin (92) in a state where the skin (92) is introduced into the recessed space (S1). The treatment efficiency for treating the skin (92) can be improved accordingly.

In a photocosmetic device (100) of a thirteenth aspect realized in combination with any one of the first to twelfth aspects, at least part of the skin introducing unit (8) is detachably attached to the housing (1).

This aspect can facilitate to illuminate the light on the skin (92) in a state where the skin (92) is introduced into the recessed space (S1). The treatment efficiency for treating the skin (92) can be improved accordingly.

In a photocosmetic device (100) of a fourteenth aspect realized in combination with any one of the first to thirteenth aspects, at least part of the skin introducing unit (8) is continuously integrated with the housing (1).

This aspect can facilitate to illuminate the light on the skin (92) in a state where the skin (92) is introduced into the recessed space (S1). The treatment efficiency for treating the skin (92) can be improved accordingly.

In a photocosmetic device (100) of a fifteenth aspect realized in combination with any one of the first to fourteenth aspects, the skin introducing unit (8) includes an assisting part (a first assisting part 87, a second assisting part 88). The assisting part is configured to, when the skin (92) is introduced into the recessed space (S1), come into contact with a part around the skin (92) introduced into the recessed space (S1).

This aspect can prevent the skin (92) from escaping (slipping out) from the recessed space (S 1).

In a photocosmetic device (100) of a sixteenth aspect realized in combination with any one of the first to fifteenth aspects, the light emitting unit (2) includes a first light source unit (201) and a second light source unit (202) each of which includes a light source (22). The housing (1) includes a first housing part (105) in which the first light source unit (201) is housed and a second housing part (106) in which the second light source unit (202) is housed. The first housing part (105) and the second housing part (106) are disposed separately from each other in a direction in which the first light source unit (201) and the second light source unit (202) are arranged.

This aspect can reduce light (leakage light) traveling from the light source (22) of one of the first light source unit (201) and the second light source unit (202) toward the light source (22) of the other.

In a photocosmetic device (100) of a seventeenth aspect realized in combination with the sixteenth aspect, at least one of the first housing part (105) or the second housing part (106) is movable in the direction in which the first light source unit (201) and the second light source unit (202) are arranged.

According to this aspect, the light exit surface (13) can be in contact with the skin (92) more closely.

A photocosmetic device (100) of an eighteenth aspect realized in combination with the seventeenth aspect further includes a stopper mechanism configured to restrict the movement of at least one of the first housing part (105) or the second housing part (106) such that a gap remains between the first housing part (105) and the second housing part (106) even in a state where the first housing part (105) and the second housing part (106) are approached closest each other.

This aspect can prevent the skin (92) from being tightly caught between the first housing part (105) and the second housing part (106). The uncomfortable feeling to be caused in a user can be reduced.

A photocosmetic device (100) of a nineteenth aspect realized in combination with any one the first to eighteenth aspects further includes an imaging unit (65). The imaging unit (65) is housed in the housing (1). The housing (1) has a recess (19). The recess (19) of the housing (1) has a bottom surface (190), and at least one side surface (191, 192) from which the light of the light emitting unit (2) is emerged outside. The imaging unit (65) has a light incident surface (650) in the bottom surface (190) of the recess (19) of the housing (1). The imaging unit (65) is configured to take an image based on light entering through the light incident surface (650).

A photocosmetic device (100) of a nineteenth aspect includes a light emitting unit (2), a housing (1), and an imaging unit (65). The light emitting unit (2) is configured to generate light to be illuminated on a skin (92). The light emitting unit (2) is housed in the housing (1). The imaging unit (65) is housed in the housing (1). The housing (1) has a recess (19). The recess (19) of the housing (1) has a bottom surface (190), and at least one side surface (191, 192) from which the light of the light emitting unit (2) is emerged outside. The imaging unit (65) has a light incident surface (650) in the bottom surface (190) of the recess (19) of the housing (1). The imaging unit (65) is configured to take an image based on light entering through the light incident surface (650).

This aspect can increase the certainty that an image taken region of the imaging unit (65) coincides with a region to which the light of the light emitting unit 2 is illuminated. This can reduce the decline in the treatment efficiency to treat the skin (92).

A photocosmetic device (100) of a twentieth aspect realized in combination with the nineteenth aspect further includes an analyzing unit (712) and a lighting controller (713). The analyzing unit (712) is configured to analyze a condition of the skin (92) based on the image taken by the imaging unit (65). The lighting controller (713) is configured to control an operation of the light emitting unit (2) based on an analysis result by the analyzing unit (712).

This aspect can facilitate to illuminate the skin (92) with light suitable for the condition of the skin (92).

In a photocosmetic device (100) of a twenty-first aspect realized in combination with the twentieth aspect, the analyzing unit (712) is configured to determine, based on the image taken by the imaging unit (65), a presence or absence of a first portion in a surface of the skin (92). The first portion is a portion having a predetermined feature. The lighting controller (713) is configured to control the operation of the light emitting unit (2) such that when the first portion is determined to be present in the surface of the skin (92), an exposure amount of the light from the light emitting unit (2) to the first portion in the surface of the skin (92) is smaller than an exposure amount of the light from the light emitting unit (2) to a second portion in the surface of the skin (92). The second portion is a portion in the surface of the skin (92) other than the first portion.

This aspect can facilitate to illuminate the skin (92) with light suitable for the skin (92) depending on a condition whether the predetermined feature is present or not in the skin (92).

A photocosmetic device (100) of a twenty-second aspect realized in combination with the nineteenth aspect further includes a communications unit (73) and a lighting controller (713). The communications unit (73) is configured to communicate with a terminal device (99) provided externally. The lighting controller (713) is configured to control an operation of the light emitting unit (2). The communications unit (73) has a function to output, to the terminal device (99), first information relating to the image taken by the imaging unit (65), and a function to receive, from the terminal device (99), second information relating to a condition of the skin (92) analyzed based on the image of the first information. The lighting controller (713) is configured to control the operation of the light emitting unit (2) based on the second information supplied from the terminal device (99).

This aspect can facilitate to illuminate the skin (92) with light suitable for the condition of the skin (92).

A photocosmetic device (100) of a twenty-third aspect realized in combination with any one of the nineteenth to twenty-second aspects further includes a lighting controller (713) configured to control an operation of the light emitting unit (2). The light emitting unit (2) includes a plurality of LEDs (221). The lighting controller (713) is configured to individually control operations of the plurality of LEDs (221).

This aspect allows a desired LED (221) selected from the plurality of LEDs (221) to emit light.

A photocosmetic device (100) of a twenty-fourth aspect realized in combination with any one of the nineteenth to twenty-third aspects further includes a cooling member and a thermal insulation part (66). The cooling member is disposed to be brought into contact with the skin (92) to cool the skin (92). The thermal insulation part (66) thermally insulates the imaging unit (65) from the cooling member (light transmission member 3).

This aspect can prevent the dew condensation water from appearing on the light incident surface (650) of the imaging unit (65).

In a photocosmetic device (100) of a twenty-fifth aspect realized in combination with any one of the nineteenth to twenty-fourth aspects, the imaging unit (65) includes an imaging device (651) including an image sensor, and at least one of an imaging light source (652) or a light guide (653). The imaging device (651) or the light guide (653) is disposed in a part of the bottom surface (190) of the recess (19) of the housing (1).

This aspect can reduce the decline in the treatment efficiency to treat the skin (92).

In a photocosmetic device (100) of a twenty-sixth aspect realized in combination with any one of the nineteenth to twenty-fifth aspects, the imaging unit (65) includes an imaging device (651) including an image sensor, and a light guide (653). The light guide (653) has the light incident surface (650) of the imaging unit (65). The imaging device (651) and the light guide (653) are arranged in an order of: the bottom surface (190) of the recess (19) of the housing (1), the light guide (653), and the imaging device (651).

This aspect can prevent the dew condensation on the surface of the imaging device (651).

In a photocosmetic device (100) of a twenty-seventh aspect realized in combination with any one of the nineteenth to twenty-sixth aspects, the imaging unit (65) includes an imaging device (651) including an image sensor. The imaging device (651) has the light incident surface

(650) of the imaging unit (65).

This aspect can increase the volume of another member (such as the heatsink) by the volume of the light guide (653).

In a photocosmetic device (100) of a twenty-eighth aspect realized in combination with any one of the nineteenth to twenty-seventh aspects, the recess (19) of the housing (1) has a recessed groove shape. The light incident surface (650) of the imaging unit (65) is located in the part of the housing (1) corresponding to the bottom surface (190) of the recess (19) to be positioned around a center in a longitudinal axis of the recess (19).

According to this aspect, an image of substantially a whole area of the recessed space can be taken by a single imaging unit (65).

A photocosmetic device (100) of a twenty-ninth aspect realized in combination with any one of the nineteenth to twenty-eighth aspects further includes a communications unit (73) configured to communicate with a terminal device (99) provided externally. The communications unit (73) has a function to output, to the terminal device (99), image information relating to the image taken by the imaging unit (65).

According to this aspect, the image taken by the imaging unit (65) can be confirmed through the terminal device (99).

A photocosmetic system (1000) of a thirtieth aspect includes the photocosmetic device (100) of the twenty-ninth aspect and the terminal device (99). The imaging unit (65) is configured to take images a plurality of times at different timings. The terminal device (99) has a function to receive, from the communications unit (73) of the photocosmetic device (100), the image information relating to a plurality of the images taken by the imaging unit (65) at the different timings, and a function to display, in time series on a display unit (992), two or more images selected from the plurality of images represented by the image information thus received.

This allows the user who sees the display of the terminal device (99) to easily understand the usefulness of the device.

Features of the second to twenty-ninth aspects are optional for the photocosmetic device (100) and may be appropriately omitted.

### Reference Signs List

- 100: Photocosmetic device
- 1: Housing
- 105: First Housing Part
- 106: Second Housing Part
- 13: Light Exit Surface
- 131: First Inclined Surface
- 132: Second Inclined Surface
- 19: Recess
- 190: Bottom Surface
- 191: Side Surface
- 2: Light Emitting Unit
- 201: First Light Source Unit
- 202: Second Light Source Unit
- 22: Light Source
- 221: LED
- 3: Light Transmission Member (Cooling Member)
- 5: Cooler
- 50: Heat Conductor
- 65: Imaging Unit
- 650: Light Incident Surface
- 651: Imaging Device
- 652: Imaging Light Source
- 653: Light Guide
- 66: Thermal Insulation Part
- 712: Analyzing Unit
- 713: Lighting Controller
- 73: Communications Unit
- 8: Skin Introducing Unit
- 80: Pinching Unit
- 800: Opening
- 81: Base Part
- 82: Protruding part
- 83: Tip End
- 84: Inclined Surface
- 86: Recessed Surface
- 87: First Assisting Part (Assisting Part)
- 88: Second Assisting Part (Assisting Part)
- 92: Skin
- 99: Terminal Device
- 992: Display Unit
- 1000: Photocosmetic System
- A1: Moving Axis
- S1: Space (Recessed Space)
- X1: Depth
- Y1: Dimension
- θ1: Crossing Angle

## Claims

1. A photocosmetic device comprising:
a light emitting unit configured to generate light to be illuminated on a skin;
a housing in which the light emitting unit is housed; and
a light exit surface from which the light of the light emitting unit is emitted to an outside of the housing,
the light exit surface having a recessed shape recessed inward the housing, and
the photocosmetic device further comprising
a skin introducing unit having an opening and configured to introduce the skin through the opening into a recessed space surrounded by the light exit surface.

2. The photocosmetic device of claim 1, wherein
the skin introducing unit includes a pinching unit configured to pinch the skin to introduce the skin into the recessed space.

3. The photocosmetic device of claim 2, wherein
the pinching unit is movable relative to the housing along a moving axis so as to change an opening size of the opening.

4. The photocosmetic device of claim 3, wherein
the pinching unit includes
a base part supported by the housing to be movable relative to the housing and
a protruding part protruding from the base part along the moving axis,
the protruding part has a tip end constituting one edge of the opening,
in an imaginal plane containing the moving axis and an opening direction of the opening, the protruding part has at least one shape selected from a group consisting of a square bracket shape, an inclined surface shape, a semicircular shape, a quarter of a circle shape, an arc shape, and a curved surface shape including two or more curves having different curvatures.

5. The photocosmetic device of claim 3, wherein
the pinching unit includes a protruding part that faces the recessed space, and
the protruding part has a recessed surface recessed away from the recessed space.

6. The photocosmetic device of any one of claims 3 to 5, wherein
the pinching unit includes
a base part supported by the housing to be movable relative to the housing and
a protruding part protruding from the base part along the moving axis,
the protruding part has a tip end constituting one edge of the opening,
when viewed in an opening direction of the opening, the tip end of the protruding part has at least one shape selected from a group consisting of a straight line shape, a comb shape, a triangle wave shape, a reverse semicircular wave shape, and a circular wave shape.

7. The photocosmetic device of any one of claims 1 to 6, further comprising a light transmission member allowing the light of the light emitting unit to pass through,
the light exit surface is at least partially constituted by a surface of the light transmission member.

8. The photocosmetic device of claim 7, wherein
the light emitting unit includes a light source disposed to face the light transmission member.

9. The photocosmetic device of any one of claims 1 to 8, wherein
at least part of the skin introducing unit is detachably attached to the housing.

10. The photocosmetic device of any one of claims 1 to 9, wherein
at least part of the skin introducing unit is continuously integrated with the housing.

11. The photocosmetic device of any one of claims 1 to 10, wherein
the skin introducing unit includes an assisting part configured to, when the skin is introduced into the recessed space, come into contact with a part around the skin introduced into the recessed space.

12. The photocosmetic device of any one of claims 1 to 11, wherein
the light emitting unit includes a first light source unit and a second light source unit each of which includes a light source,
the housing includes a first housing part in which the first light source unit is housed and a second housing part in which the second light source unit is housed,
the first housing part and the second housing part are disposed separately from each other in a direction in which the first light source unit and the second light source unit are arranged.

13. The photocosmetic device of claim 12, wherein at least one of the first housing part or the second housing part is movable in the direction in which the first light source unit and the second light source unit are arranged.

14. The photocosmetic device of claim 13, further comprising a stopper mechanism configured to restrict a movement of at least one of the first housing part or the second housing part such that a gap remains between the first housing part and the second housing part even in a state where the first housing part and the second housing part are approached closest each other.

15. The photocosmetic device of any one of claims 1 to 14, further comprising an imaging unit housed in the housing, wherein
the housing has a recess, the recess of the housing having a bottom surface and at least one side surface from which the light of the light emitting unit is emerged outside,
the imaging unit has a light incident surface in the bottom surface of the recess of the housing, the imaging unit being configured to take an image based on light entering through the light incident surface.

16. The photocosmetic device of claim 15, further comprising:
an analyzing unit configured to analyze a condition of the skin based on the image taken by the imaging unit; and
a lighting controller configured to control an operation of the light emitting unit based on an analysis result by the analyzing unit.

17. The photocosmetic device of claim 16, wherein
the analyzing unit is configured to determine, based on the image taken by the imaging unit, a presence or absence of a first portion having a predetermined feature in a surface of the skin, and
the lighting controller is configured to control the operation of the light emitting unit such that when the first portion is determined to be present in the surface of the skin, an exposure amount of the light from the light emitting unit to the first portion in the surface of the skin is smaller than an exposure amount of the light from the light emitting unit to a second portion in the surface of the skin, wherein the second portion is a portion in the surface of the skin other than the first portion.

18. The photocosmetic device of claim 15, further comprising:
a communications unit configured to communicate with a terminal device provided externally; and
a lighting controller configured to control an operation of the light emitting unit, wherein
the communications unit has
a function to output, to the terminal device, first information relating to the image taken by the imaging unit, and
a function to receive, from the terminal device, second information relating to a condition of the skin analyzed based on the image of the first information, and
the lighting controller is configured to control the operation of the light emitting unit based on the second information supplied from the terminal device.

19. The photocosmetic device of any one of claims 15 to 18, further comprising a lighting controller configured to control an operation of the light emitting unit, wherein
the light emitting unit includes a plurality of LEDs, and
the lighting controller is configured to individually control operations of the plurality of LEDs.

20. The photocosmetic device of any one of claims 15 to 19, further comprising:
a cooling member disposed to be brought into contact with the skin to cool the skin; and
a thermal insulation part thermally insulating the imaging unit from the cooling member.

21. The photocosmetic device of any one of claims 15 to 20, wherein
the imaging unit includes
an imaging device including an image sensor and
at least one of an imaging light source or a light guide, and
the imaging device or the light guide is disposed in a part of the bottom surface of the recess of the housing.

22. The photocosmetic device of any one of claims 15 to 21, wherein
the imaging unit includes
an imaging device including an image sensor and
a light guide,
the light guide has the light incident surface of the imaging unit,
the imaging device and the light guide are arranged in an order of: the bottom surface of the recess of the housing; the light guide; and the imaging device.

23. The photocosmetic device of any one of claims 15 to 21, wherein
the imaging unit includes an imaging device including an image sensor, and
the imaging device has the light incident surface of the imaging unit.

24. The photocosmetic device of any one of claims 15 to 23, wherein
the recess of the housing has a recessed groove shape,
the light incident surface of the imaging unit is located in the part of the housing corresponding to the bottom surface of the recess to be positioned around a center in a longitudinal axis of the recess.

25. The photocosmetic device of any one of claims 15 to 24, further comprising a communications unit configured to communicate with a terminal device provided externally, wherein
the communications unit has a function to output, to the terminal device, image information relating to the image taken by the imaging unit.

26. A photocosmetic system, comprising:
the photocosmetic device of claim 25; and
the terminal device, wherein
the imaging unit is configured to take images a plurality of times at different timings, and
the terminal device has
a function to receive, from the communications unit of the photocosmetic device, the image information relating to a plurality of the images taken by the imaging unit at the different timings, and
a function to display, in time series on a display unit, two or more images selected from the plurality of images represented by the image information thus received.
